# EUROPEAN PATENT SPECIFICATION

(11) **EP 2 114 406 B1**
(45) Date of publication and mention of the grant of the patent: **25.05.2011**
(21) Application number: 08700129.3
(22) Date of filing: 07.01.2008
(51) Int. Cl.: A61K 31/496, A61P 31/04, A61P 33/00

(54) **THIOXANTHENE DERIVATES AS SOLE ANTI-INFECTIVE AGENTS FOR USE IN THE TREATMENT OF INFECTIOUS DISEASES**
THIOXANTHEN-DERIVATE ALS ALLEINVERTRETER DER ANTIINFEKTIVA ZUR VERWENDUNG IN DER BEHANDLUNG VON INFEKTIONSKRANKHEITEN
DÉRIVÉS DE THIOXANTHÈNE COMME SEULS AGENTS ANTIINFECTIEUX POUR LEUR UTILISATION DANS LE TRAITEMENT DES MALADIES INFECTIEUSES

(30) Priority: 05.01.2007 WO PCT/DK2007/000006
(43) Date of publication of application: 11.11.2009
(60) Divisional application: 10181816.9
(73) Proprietor: BKG Pharma ApS, 2920 Charlottenlund (DK)
(72) Inventor: GIWERCMAN, Birgit, Kjælgaard, DK-2920 Charlottenlund (DK)
(74) Representative: Kitchen, Steven Richard
(86) International application number: PCT/DK2008/000005
(87) International publication number: WO 2008/080408

(56) References cited:
- EP-A- 0 338 532
- WO-A-2005/105145
- US-A1- 2002 151 543
- MORTENSEN I ET AL: "THE ANTIBACTERIAL ACTIVITY OF THE PSYCHOPHARMACOLOGICAL AGENT CLOPENTHIXOL AND ITS TWO MAIN METABOLITES" ACTA PATHOLOGICA MICROBIOLOGICA SCANDINAVICA, SECTION B, XX, XX, vol. 95B, no. 6, 1987, pages 355-359, XP008066133 ISSN: 0108-0180
- KOCISKO ET AL: "Comparison of protease-resistant prion protein inhibitors in cell cultures infected with two strains of mouse and sheep scrapie" NEUROSCIENCE LETTERS, LIMERICK, IE, vol. 388, no. 2, 11 November 2005 (2005-11-11), pages 106-111, XP005042190 ISSN: 0304-3940
- KAISER C ET AL: "Analogs of phenothiazines. 4. Effect of structure upon neuropharmacological activity of some chlorpromazine analogs of the diphenylmethane type." JOURNAL OF MEDICINAL CHEMISTRY JUN 1972, vol. 15, no. 6, June 1972 (1972-06), pages 665-673, XP007904473 ISSN: 0022-2623
- JEYASEELI L ET AL: "Antimicrobial potentiality of the thioxanthene flupenthixol through extensive in vitro and in vivo experiments" INTERNATIONAL JOURNAL OF ANTIMICROBIAL AGENTS, ELSEVIER SCIENCE, AMSTERDAM, NL LNKD- DOI:10.1016/J.IJANTIMICAG.2005.08.014, vol. 27, no. 1, 1 January 2006 (2006-01-01), pages 58-62, XP025082133 ISSN: 0924-8579 [retrieved on 2006-01-01]

## Description

### Technical Field

The present invention is directed to the use of anti-infective agents, in particular thioxanthene derivatives and phenothiazine derivatives, for the manufacture of a medicament for the treatment of infectious diseases.

### Background

Resistance to chemotherapy is a common clinical problem in patients with infectious diseases. During treatment of infections the drug targets of prokaryotic or eukaryotic microorganisms cells are often found to be refractory to a variety of drugs that have different structures and functions. This phenomenon has been termed multidrug resistance (MDR).

The incidence of the multiple antimicrobial resistance of bacteria which cause infections in hospitals/intensive care units is increasing, and finding microorganisms insensitive to more than 10 different antibiotics is not unusual. Examples of such resistant bacteria include methicillin-resistant and methicillin-vancomycin-resistant *Staphylococcus aureus*; vancomycin-resistant enterococci, such as *Enterococcus faecalis* and *Enterococcus faecium;* penicillin-resistant *Streptococcus pneumoniae,* and cephalosporin and quinolone resistant gram-negative rods (coliforms), such as *E*. *coli, Salmonella* species, *Klebsiella pneumoniae, Pseudomonas* species and *Enterobacter* species. More recently, pan antibiotic resistant gram-negative and gram-positive bacilli have emerged.

The rapidity of emergence of these multiple antibiotic-resistance is not being reflected by the same rate of development of new antibiotics and it is, therefore, conceivable that patients with serious infections soon will no longer be treatable with currently available anti-infective agents. Several international reports have highlighted the potential problems associated with the emergence of antimicrobial resistance in many areas of medicine and also outlined the difficulties in the management of patients with infections caused by these microorganisms.

Although most of the hardier microorganisms are present in hospitals, strains of multidrug resistant bacteria, such as *Streptococcus pneumoniae* and *Mycobacterium tuberculosis* have also caused serious community-acquired infections. The prevalence of drug-resistant *Streptococcus pneumoniae* has increased 60-fold since 1980 with 51% and 8% of isolates demonstrating intermediate- or high-level resistance to penicillin or third-generation cephalosporins, respectively. Thus, pneumococcal pneumonia is becoming more difficult to treat with first-line anti-infective agents. Resistant bacteria from hospitals can be introduced into the community via patients discharged for continued treatment at home taking with them, for example, multidrug resistant *Staphylococcus aureus* and vancomycin resistant enterococci.

Phenothiazines have been shown to be among the group of drugs known to modify resistance to one or more antibacterial agents in certain bacteria. Phenothiazines and thioxanthenes are used clinically as neuroleptic and antiemetic agents. Phenothiazines, and structurally related antipsychotic agents, inhibit several cellular enzymes and block the function of critical cellular receptors. The extrapyramidal side effects associated with antipsychotic therapy are attributed to dopamine receptor binding. In general these extrapyramidal side effects have proven to be dose limiting in clinical trials using phenothiazines and thioxanthenes in non-psychotic areas, such as anti-cancer treatment.

Although the mechanism by which phenothiazines and other drugs modulate MDR is not yet clear, it has been suggested that their pharmacological properties may be mediated at least in part by inhibition of efflux pumps. Also, promethazine has been recognised as an effective antiplasmid agent in cultures containing bacterial species such as *Escherichia coli, Yersinia enterocolitica, Staphylococcus aureus and Agrobacterium tumefaciens.* The concentrations used, however, are generally high above clinically relevant concentrations.

It has recently been shown that certain phenothiazine and thioxanthene derivatives used as anti-infective compounds are surprisingly effective in assisting in killing infectious agents, such as multidrug resistant infectious agents, even at clinically relevant concentrations, when used in combination with an anti-infective agent. Accordingly, WO05105145 disclose the use of certain thioxanthene derivatives and phenothiazine derivatives as anti-infectious compounds for the treatment of infectious disease in combination with an anti-infectious agent.

Several phenothiazine and thioxanthene derivatives are disclosed in US 6,569,853.

Flupenthixol is disclosed in UK Patent 925,538 as having utility as tranquilliser, ataractic, antiemetic, antihistamine, antispasmodic and general central nervous system depressant. No mention is made of any anti-infective or anti-resistance activity.

Several thioxanthene derivatives are disclosed in UK Patent 863,699 as tranquillisers. No mention is made of any of anti-infective or anti-resistance activity.

WO2005/105145 A discloses the use of certain phenothiazines and thioxanthenes e.g. flupenthixol and clopenthixol as antibacterial agents. The problem solved relates to combination treatment of infective diseases and the teaching relating to this disclosure is that the disclosed compounds are not suited for administration as single antibacterial agents but rather that the disclosed compounds are suited for combination treatment where another antibiotic agent is used simultaneously in combination with the disclosed compounds. The present invention differ in this respect by solving another problem by the provision of novel compounds (compounds which are among the generally disclosed compounds of WO2005/105145), that surprisingly have been found to be suited for administration as anti infective agents alone.

EP-A-0338532 discloses the use of clopenthixol among other compounds as an anti-protozoal agent.

Kolaczlsowski M et al., International Journal of Antimicrobial Agents (2003) vol 2, nr. 3 discloses trans-flupenthixol among a range of compounds as modulators of yeast multidrug resistance.

Kristensen et al., International Journal of Antimicrobial Agents (2000) vol 14, nr. 3 discloses cis- and trans-flupenthixol as HIV-inhibitors.

Phenothiazines and thioxanthenes have been shown in themselves to have modest, but broad, antimicrobial activities. MICs (the minimal concentration of compound at which the infectious agent is inhibited) are generally high above clinically relevant concentrations inasmuch as the disclosed minimum effective concentrations *in vitro* are in the order from approximately 20 mg/l to several hundreds mg/l. The relevant serum levels of phenothiazines and thioxanthenes are generally in the range from approximately 0.3 µg/l to 0.5 mg/l (0.3 ng/ml to 0.5 µg/ml) in order to avoid potential side effects.

The thioxanthenes demonstrate geometric stereoisomerism. The *cis* and *trans* forms have previously been shown to have roughly equal modest antibacterial potency. MICs are generally far above clinically relevant concentrations.

Mortensen & Kristiansen (Acta Pathologica Microbiologica Scandinavia, section B, 95B, no. 6 (1987) showed that a cis/trans mixture of N-dealkyl-clopenthixol had a modest inhibitory effect on different laboratory bacterial strains. The bacterial strains were not resistant to anti-infective agents (i.e. they did not have any acquired resistance and are described as being "sensitive"). The inhibitory effect was measured as IC50 values, which give the concentrations at which 50% of the population of a single bacterial strain is inhibited. The authors state that the cis/trans mixture of N-dealkyl-clopenthixol was not able to inhibit 50% of the gram-negative strains tested, even at a concentration of 100µM (59,7µg/ml). The authors further state that a concentration of 12,5 µg/ml of the cis/trans mixture of N-dealkyl-clopenthixol was required for all the gram-positive strains tested to be inhibited, measured by IC50- The calculation of IC50 is based on a semi-quantitation of the size of colonies growing on plates made by the naked eye. This method is not approved by NCCLS or European guidelines. Mortensen & Kristiansen is silent on the minimal inhibitory concentration (MIC) of the compounds. The Minimal Inhibitory Concentration (MIC) is an internationally approved measure of anti-infectiveness of compounds. MIC is defined as the lowest inhibitory concentration showing no visible growth according to the NCCLS Guidelines. IC50 concentrations are in most cases, if not all, several fold lower than the corresponding MIC value. According to a publication by Kristensen in Danish Medical Bulletin Vol 37 No 2/april 1990, based on the results of e.g. the above mentioned study by Mortensen & Kristiansen the concentrations required for use as antibiotic is 100-1000 times the concentrations measured to be tolerated without side effects in humans. As seen on page 170 the author state that "*the concentrations of psychotherapeutic drugs employed in the in vitro experiments (I, II, III, IV, V, VI, VII and VIII) are at least 100-1000 times higher than the plasma concentrations of free drug that can be measured in psychiatric patients under treatment".* This leads the author to conclude on page 176 that " *..an evaluation of the in-vitro-experiments with trans-clopenthixol treatment of mice infected with pneumococci shows that is difficult to conceive how therapeutic doses of known psychotherapeutic drugs and their analogues alone could be used as antibiotic drugs"*

These reportings by Kristiansen et al., that very high levels of compound is apparently needed in order to inhibit bacterial growth, even measured by IC50, has probably caused a lack of interest and research in the use of this type of phenothiazine and thioxanthene derivatives for treatment of infectious disease.

It is clear that the increase in resistance to anti-infective agents, such as antibiotics, present a major impediment to the treatment of infections. Thus, there is an urgent need for new anti-infective agents.

The object of the present invention is to provide anti-infective agents capable of killing or inhibiting growth of clinically relevant miroorganisms, especially resistant, including multidrug resistant, cells or microorganisms by administration of clinically relevant amounts of such anti-infective agents to a subject in need thereof.

### Disclosure of the Invention

As will be understood from the above discussion, the prior art has hitherto deemed thioxanthenes and phenothiazines unsuitable for treatment of infections as sole anti-infective agent, since the necessary therapeutic amount of such anti-infective agents would cause severe side effects.

Thus, despite the prejudice in the art that thioxanthene and phenothiazine concentrations far above the clinical relevant level would be necessary in order to combat microorganisms, in particular resistant or multidrug resistant microorganisms, as sole anti-infective agent the present inventor decided to investigate this matter in further detail by studying the effect of such anti-infective agent on clinically relevant isolates, including resistant and multidrug resistant strains, using clinically relevant amounts of the anti-infective agents described herein. Further the inventor investigated the steroisomeric influence on activity.

Surprisingly, it was found that by applying clinically relevant amounts of the anti-infective agents described herein, effective killing of microorganisms, including resistant or multidrug resistant clinically relevant isolates was achieved. Contrary to what was previously believed, this surprising finding opens up the possibility for effectively combating microorganisms by use of the anti-infective agents described herein as sole anti-infective agent.

Accordingly, in a first aspect the present invention relates to an anti-infective agent of the general formula (I) 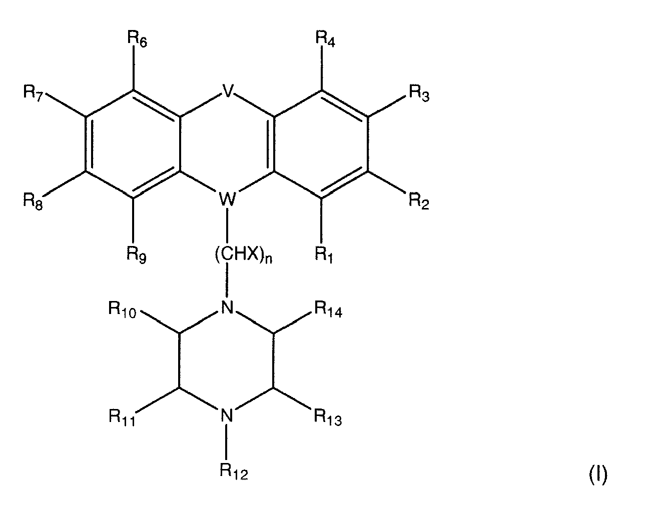 wherein
V is selected from the group consisting of S, SO₂, SO, O and NH;
W is C=CH;
n is an integer in the range of from 1 to 6;
each X is individually selected from the group consisting of hydrogen, halogen, hydroxy, amino, nitro, optionally substituted C₁₋₆-alkyl and optionally substituted C₁₋₆-alkoxy;
R₁, R₂, R₃, R₄, R₅, R₆, R₇, R₈, R₉, R₁₀, R₁₁, R₁₃ and R₁₄ are each individually selected from the group consisting of hydrogen, halogen, hydroxy, amino, nitro, optionally substituted C₁₋₆-alkyl, optionally substituted C₂₋₅-alkenyl, optionally substituted C₂₋₆-alkynyl and optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, optionally substituted C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkyl-carbonylamino, mono- and di(C₁₋₆-alkyl)amino-C₁₋₆-alkyl-carbonylamino, amino-C₁₋₆-alkyl-amino, mono- and di(C₁₋₆-alkyl)amino-C₁₋₆-alkyl-amino, cyano, guanidino, carbamido, C₁₋₆-alkanoyloxy, C₁₋₆-alkylsulphonyl, C₁₋₆-alkylsulphinyl, C₁₋₆-alkylsulphonyloxy, aminosulfonyl, mono- and di(C₁₋₆-alkyl)aminosulfonyl, and optionally substituted C₁₋₆-alkylthio; and
R₁₂ is hydrogen, halogen, hydroxy, amino, nitro, CH₂Y, CHY₂ and CY₃, wherein each Y is individually selected among hydrogen, hydroxy, amino, nitro or halogen;
or a salt thereof for use in the treatment or prophylaxis of an infectious disease, caused by an infections agent selected among pathogenic bacteria, fungi and vira, wherein the anti-infective agent is used as sole, anti-infective agent. The invention further relates to the use of the agent according to the invention for the manufacture of a medicament for treatment or prophylaxis of an infectious disease, caused by an infections agent selected among pathogenic bacteria, fungi and vira, wherein the anti-infective agent is used as sole, anti-infective agent.

Other aspect of the present invention will be apparent from the below description and the appended claims.

### Detailed description of the Invention

### Definitions

In the present context, the term "C₁₋₆-alkyl" is intended to mean a linear or branched saturated hydrocarbon group having from one to six carbon atoms, such as methyl, ethyl, n-propyl, isopropyl, n-butyl, isobutyl, *sec*-butyl, *tert*-butyl, n-pentyl, isopentyl, neopentyl and n-hexyl.

In the present context the term "C₃₋₆-cycloalkyl" is intended to cover three-, four-, five- and six-membered rings comprising carbon atoms only, whereas the term "heterocyclyl" is intended to mean three-, four-, five- and six-membered rings wherein carbon atoms together with from 1 to 3 heteroatoms constitute said ring. The heteroatoms are independently selected from oxygen, sulphur, and nitrogen. C₃₋₆-cycloalkyl and heterocyclyl rings may optionally contain one or more unsaturated bonds situated in such a way, however, that an aromatic π-electron system does not arise.

Illustrative examples of "C₃₋₆-cycloalkyl" are the carbocycles cyclopropane, cyclobutane, cyclopentane, cyclopentene, cyclopentadiene, cyclohexane, cyclohexene, 1,3-cyclohexadiene and 1,4-cyclohexadiene.

Illustrative examples of "heterocyclyls" are the nitrogen-containing heterocycles 2-pyrrolinyl, 3-pyrrolinyl, pyrrolidinyl, 2-imidazolinyl, imidazolidinyl, 2-pyrazolinyl, 3-pyrazolinyl, pyrazolidinyl, piperidinyl, morpholinyl, thiomorpholinyl and piperazinyl, Binding to the heterocycle may be at the position of the heteroatom or via a carbon atom of the heterocycle.

In the present context, the term "C₂₋₆-alkenyl" is intended to mean a linear or branched hydrocarbon group having from two to six carbon atoms and containing one or more double bonds. Illustrative examples of C₂₋₆-alkenyl groups include allyl, homo-allyl, vinyl, crotyl, butenyl, pentenyl and hexenyl. Illustrative examples of C₂₋₆-alkenyl groups with more than one double bond include butadienyl, pentadianyl and hexadienyl. The position of the double bond(s) may be at any position along the carbon chain.

In the present context the term "C₂₋₆-alkynyl" is intended to mean a linear or branched hydrocarbon group containing from two to six carbon atoms and containing one or more triple bonds. Illustrative examples of C₂₋₆-alkynyl groups include acetylene, propynyl, butynyl, pentynyl and hexynyl. The position of the triple bond(s) may be at any position along the carbon chain. More than one bond may be unsaturated so that the "C₂₋₆-alkynyl" is a di-yne or enedi-yne as is known to the person skilled in the art. When used herein the term "C₁₋₆-alkoxy" is intended to mean C₁₋₆-alkyl-oxy, such as methoxy, ethoxy, n-propoxy, isopropoxy, n-butoxy, isobutoxy, *sec*-butoxy, *tert-*butoxy, n-pentoxy, isopentoxy, neopentoxy and n-hexoxy.

The term "halogen" includes fluorine, chlorine, bromine and iodine.

In the present context the term "aryl" is intended to mean a carbocyclic aromatic ring or ring system. Moreover, the term "aryl" includes fused ring systems wherein at least two aryl rings, or at least one aryl and at least one C₃₋₆-cycloalkyl, or at least one aryl and at least one heterocyclyl, share at least one chemical bond. Illustrative examples of "aryl" rings include phenyl, naphthalenyl, phenanthrenyl, anthracenyl, acenaphthylenyl, tetralinyl, fluorenyl, indenyl, indolyl, coumaranyl, coumarinyl, chromanyl, isochromanyl, and azulenyl.

In the present context, the term "heteroaryl" is intended to mean an aryl group where one or more carbon atoms in an aromatic ring have been replaced with one or more heteroatoms selected from the group consisting of nitrogen, sulphur, phosphorous and oxygen. Furthermore, in the present context, the term "heteroaryl" comprises fused ring systems wherein at least one aryl ring and at least one heteroaryl ring, at least two heteroaryls, at least one heteroaryl and at least one heterocyclyl, or at least one heteroaryl and at least one C₃₋₆-cycloalkyl share at least one chemical bond.

Illustrative examples of a heteroaryl include furanyl, thienyl, pyrrolyl, phenoxazonyl, oxazolyl, thiazolyl, isothiazolyl, imidazolyl, pyrazolyl, isoxazolyl, imidazolyl isothiazolyl, oxadiazolyl, furazanyl, triazolyl, thiadiazolyl, piperidinyl, pyridinyl, pyridazinyl, pyrimidinyl, pyrazinyl, pyrazolyl and triazinyl, isoindolyl, indolinyl, benzofuranyl, benzothiophenyl, benzopyrazolyl, indazolyl, benzimidazolyl, benzthiazolyl, purinyl, quinolizinyl, quinolinyl, isoquinolinyl, cinnolinyl, phthalazinyl, quinazolinyl, quinoxalinyl, naphthyridinyl, pteridinylthienofuranyl, carbazolyl, acridinyl, phenazinyl, phenothiazinyl, phenoxazinyl and thianthrenyl.

In the present context the term "optionally substituted" is intended to mean that the group in question may be substituted one or several times, such as 1 to 5 times, preferably 1 to 3 times, most preferably 1 to 2 times, with one or more groups selected from the group consisting of C₁₋₆-alkyl, C₁₋₆-alkoxy, oxo (which may be represented in the tautomeric enol form), carboxyl, amino, hydroxy (which when present in an enol system may be represented in the tautomeric keto form), nitro, sulphono, sulphanyl, C₁₋₆-carboxyl, C₁₋₆-alkoxycarbonyl, C₁₋₆-alkylcarbonyl, formyl, aryl, aryloxy, aryloxycarbonyl, arylcarbonyl, heteroaryl, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, cyano, guanidino, carbamido, C₁₋₆-alkanoyloxy, C₁₋₆-alkylsulphonyloxy, dihalogen-C₁₋₆-alkyl, trihalogen-C₁₋₆-alkyl and halogen, where aryl and heteroaryl substituents may themselves be substituted 1-3 times with C₁₋₆-alkyl, C₁₋₆-alkoxy, nitro, cyano, hydroxy, amino or halogen. In general, the above substituents may be susceptible to further optional substitution.

The term "infectious agent" is intended to mean pathogenic microorganisms such as bacteria, fungi and vira. In a more preferred aspect of the invention the term "infectious agent" is intended to mean only pathogenic bacteria, fungi and vira. In one aspect of the invention the term "infectious agent" is intended to mean only pathogenic bacteria. In one aspect of the invention the term "infectious agent" is intended to mean only pathogenic fungi. In one aspect of the invention the term "infectious agent" is intended to mean only pathogenic vira.

Analogously, the term "infectious disease" is used about a disease caused by an infectious agent.

In the present context, the term "anti-infective agent" covers agents that are capable of killing, inhibiting or otherwise slowing the growth of the infectious agent. In a preferred aspect of the invention the term "anti-infective agent" covers agents that are capable of killing, inhibiting or otherwise slowing the growth of the infectious agent when administered to a subject in amounts that do not exceed 20 mg/l. The term "anti-infective agent" thus covers agents that exhibit a MIC value of equal to or less than 20 µg/ml when determined as described in the examples herein. The term "anti-infective agent" may be used interchangeably with the term "antibiotic" or "anti-viral agent" or "anti-fungal agent" depending on the nature of the infectious agent. Specific examples of antibiotics commonly used for treating bacterial and fungal infections include, but is not limited to, aminoglycosides, such as amikacin, gentamicin, kanamycin, neomycin, netilmicin, streptomycin and tobramycin; cabecephems, such as loracarbef; carbapenems, such as ertapenem, imipenem/cilastatin and meropenem; cephalasporins, such as cefadroxil, cefazolin, cephalexin, cefaclor, cefamandole, cephalexin, cefoxitin, cefprozil, cefuroxime, cefixime, cefdinir, cefditoren, cefoperazone, cefotaxime, cefpodoxime, ceftazidime, ceftibuten, ceftizoxime, ceftriaxone and cefepime; macrolides, such as azithromycin, clarithromycin, dirithromycin, erythromycin and troleandomycin; monobactam; penicillins, such as amoxicillin, ampicillin, carbenicillin, cloxacillin, dicloxacillin, nafcillin, oxacillin, penicillin G, penicillin V, piperacillin and ticarcillin; polypeptides, such as bacitracin, colistin and polymyxin B; quinolones, such as ciprafloxacin, enoxacin, gatifloxacin, levofloxacin, lomefloxacin, moxifloxacin, norfloxacin, ofloxacin and trovafloxacin; sulfonamides, such as mafenide, sulfacetamide, sulfamethizole, sulfasalazine, sulfisaxazole and trimethoprim-sulfamethoxazole; tetracyclines, such as demeclocycline, doxycycline, minocycline, oxytetracycline and tetracycline;

Specific examples of anti-viral agents commonly used for treating viral infections include, but is not limited to, acyclovir, amantadine, cidofovir famciclovir, fomivirsen, foscamet, ganciclovir, interferon alpha, oseltamivir, penciclovir, ribavirin, rimantadine, trifluridine, valacyctovir, valganciclovir, vidarabine and zanamivir:

Specific examples of anti-fungal agents commonly used for treating severe fungal infections include, but is not limited to, amphotericin B, caspofungin, fluconazole, flucytosine, itraconazole, ketoconazole and voriconazole.

In the present context, an infectious agent is said to be "resistant" or "drug resistant" if the infectious agent has undergone a change which reduces or eliminates the effectiveness of an anti-infective agent which is normally used to cure infections caused by the infectious agent. Analogously, the term "drug resistance" means a circumstance when a disease, e.g. an infectious disease, does not respond to a therapeutic agent, such as an anti-infective agent. Drug resistance can be intrinsic, which means that the disease has never been responsive to the therapeutic agent, or acquired, which means that the disease ceases responding to the therapeutic agent to which the disease had previously been responsive.

In the present context, an infectious agent is said to be "multidrug resistant" if the infectious agent has undergone a change which reduces or eliminates the effectiveness of two or more anti-infective agents which are normally used to cure infections caused by the infectious agent. Analogously, "multidrug resistance" is a type of drug resistance wherein a disease, e.g. an infectious disease, is resistant to a variety of drugs, such as a variety of anti-infective agents.

The term "clinically relevant amount" is intended to mean that the anti-infective agent is administered to a patient in an amount, which, on the one hand, is capable of reducing the symptoms of the infectious disease or curing the infectious disease for which the patient is treated, but, on the other hand, is not toxic to the patient and does not lead to unacceptable side effects: As indicated above, many, if not all, of the anti-infective agents described herein are known to cause severe side effects in patients when administered in too high concentrations, i.e. in amounts which are not "clinically relevant".

In the present context, the term "naturally occurring" when used in connection with the term "infectious agent", i.e. in connection with pathogenic microorganisms, means that the infectious agent giving rise to the infectious disease is a microorganism that can be found in nature, including in human beings. It will be understood that infectious agents, such as gen-manipulated laboratory strains, or infectious agents which by other means have been changed and/or manipulated by human intervention, are not considered to be covered by the term "naturally occurring".

The term "serum" is used in its normal meaning, i.e. as blood plasma without fibrinogen and other clotting factors.

Herein, the term "steady state serum concentration" (of a anti-infective agent) is defined as those values of free non-bound drug that recur with each dose and represent a state of equilibrium between the amount of anti-infective agent administered and the amount being eliminated in a given time interval.

In the present context, the term "treatment" refers to the administration of a drug to a subject and includes *i*) preventing an infectious disease (i.e. causing the clinical symptoms of the infectious disease not to develop), *ii*) inhibiting an infectious disease (i.e. arresting the development of the clinical symptoms of the infectious disease) and *iii)* relieving the disease (i.e. causing regression of the clinical symptoms of the infectious disease) as well as combinations thereof.

The terms "prophylaxis" or "prophylactic treatment" refers to the treatment of a subject who is not yet infected, but who may be susceptible to, or at risk of getting an infection.

The term "subject", as used herein, means a living vertebrate animal, e.g., a mammal, such as a human being.

"Pharmaceutically acceptable" means suitable for use in a mammal, in particular suitable for use in a human being.

### Anti-infective agents

Concerning the general formula (I) above, the substituents R₁, R₂, R₃, F_{4,} R₅, R₆, R₇, R_{8,} R₉, R₁₀, R₁₁, R₁₃ and R₁₄ are each individually selected from the group consisting of hydrogen, halogen, hydroxy, amino, nitro, optionally substituted C₁₋₆-alkyl, optionally substituted C₂₋₆-alkenyl, optionally substituted C₂₋₆-alkynyl and optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, optionally substituted C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-_{C1-6}-alkyl-aminocarbonyl, C₁₋₆-alkylcarbonylamino, amino-C₁₋₆-alkyl-carbonylamino, mono- and di(C₁₋₆-alkyl)amino-C₁₋₆-alkyl-carbonylamino, amino-C₁₋₆-alkyl-amino, mono- and di(C₁₋₆-alkyl)amino-C₁₋₆-alkylamino, cyano, guanidino, carbamido, C₁₋₆-alkanoyloxy, C₁₋₆-alkylsulphonyl, C₁₋₆-alkylsulphinyl, C₁₋₆-alkylsulphonyloxy, aminosulfonyl, mono- and di(C₁₋₆-alkyl)aminosulfonyl, and optionally substituted C₁₋₆-alkylthio.

R₁₂ is hydrogen, hydroxy, amino, nitro, halogen, CH₂Y, CHY₂ and CY₃, wherein each Y is individually selected among hydrogen, hydroxy, amino, nitro or halogen.

In a preferred embodiment of the invention, R₁₂ is selected from the group consisting of hydrogen, CH₃ and CH₂OH.

In a more preferred embodiment of the invention, R₁₂ is selected from the group consisting of hydrogen and CH₃.

In a most preferred embodiment R₁₂ is hydrogen.

In a preferred embodiment of the invention, the R₂ substituent is an electron-withdrawing group, such as halogen, nitro or halogen-substituted C₁₋₆-alkyl. More preferably, R₂ is selected from the group consisting of F, Cl, Br, I, CH₂Y, CHY₂ and CY₃ (wherein Y represents a halogen atom), such as CH₂Cl, CH₂F, CHCI₂, CHF₂, CCI₃ or CF₃, in particular CCl₃ or CF₃. Most preferably, R₂ is Cl or CF₃.

The substituents R₁, R₃, R₄, R₅, R₆, R₇, R₈, R₉, R₁₀, R₁₁, R₁₃ and R₁₄ are preferably each individually selected from the group consisting of hydrogen, optionally substituted C₁₋₆-alkyl and optionally substituted C₁₋₆-alkoxy. More preferably, all of R₁, R₃, R₄, R₅, R₆, R₇, R₈, R₉, R₁₀, R₁₁, R₁₃ and R₁₄ are hydrogen.

Accordingly, in a highly preferred embodiment of the invention, R₂ is Cl or CF₃ and each of R₁, R₃, R₄, R₅, R₆, R₇, R₈, R₉, R₁₀, R₁₁, R₁₃ and R₁₄ are hydrogen.

As mentioned above, V is selected from the group consisting of S, SO₂, SO, O and NH, such as S or SO. In a highly preferred embodiment of the invention, V is S.

As will also be understood, in case W is C=CH and n is an integer in the range of from 1 to 5, and V is S, the anti-infective agent of the general formula (I) becomes a thioxanthene of the general formula (III):

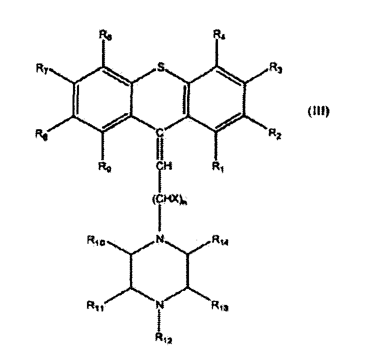

A thioxanthene of the general formula (III) gives rise to *cis* and *trans* isomerism. In the present context, agents of the general formula (IIIa) are said to be in the *cis* configuration (where the piperazinyl group is to be interpreted as being on the same side of the double bond as the part of the molecule containing the R₂ group), whereas agents of the general formula (IIIb) are said to be in the *trans* configuration (where the piperazinyl group is to be interpreted being on the opposite side of the double bond as the part of the molecule containing the R₂ group): 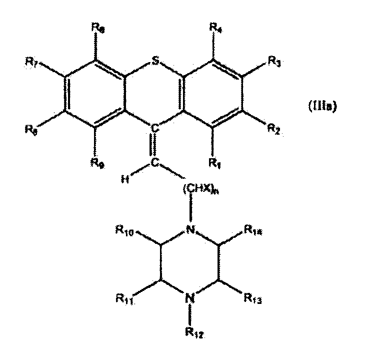 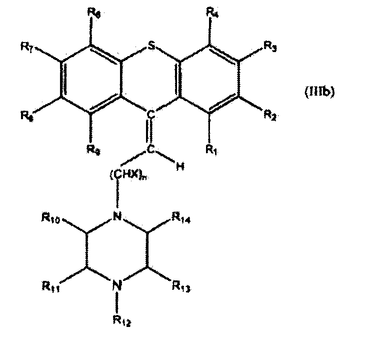 wherein n is an integer in the range of from 1 to 5, such as 1, 2, 3, 4, or 5, and each X is individually selected from the group consisting of hydrogen, halogen, hydroxy, amino, nitro, optionally substituted C₁₋₆-alkyl and optionally substituted C₁₋₆-alkoxy.

In a preferred embodiment of the invention n is 2 or 3, X is hydrogen or CH₃ and R₁₂ is hydrogen or CH₃. Particularly, it has been shown that when n is 2, and each X is hydrogen and R₁₂ is hydrogen or CH₃, the agents of the general formula (IIIa) and (IIIb) show a potent anti-infective activity at clinically relevant concentrations. Thus in a preferred embodiment of the invention, W together with the functional group attached thereto form an alkenyl chain (C=C-(CHX)ₙ-) with an optionally substituted piperazinyl group. The piperazinyl group is preferably unsubstituted or substituted in the para position (R₁₂). Thus, in a preferred embodiment W together with the functional group attached thereto is CCH-(CH₂)₂-4-methyl-piperazinyl, CCH-CH₂-CH(CH₃)-4-methylpiperazinyl, CCH-(CH₂)₂-piperazinyl or CCH-CH₂-CH(CH₃)-piperazinyl. In particular, the structure where W together with the functional group attached thereto is CCH-(CH₂)₂-4-methyl-piperazinyl is preferred.

Specific examples of thioxanthenes according to the invention include N-dealkyl-flupenthixol (4-[3-(2-(trifluoromethyl)thioxanthen-9-ylidene)propyl]1-piperazine), N-dealkyl-clopenthixol (4-[3-(2-chlorothioxanthen-9-ylidene)propyl]1-piperazine), 4-[3-(2-(trifluoromethyl)thioxanthen-9-ylidene)propyl]1-methylpiperazine, 4-[3-(2-chlorothioxanthen-9-ylidene)propyl]1-methylpiperazine. Particularly preferred anti-infective agents for the use according to the invention are N-dealkyl-flupenthixol and N-dealkyl-clopenthixol. Most preferred is N-dealkyl-clopenthixol.

Surprisingly, it has been shown that the thioxanthene anti-infective agents of the present invention are increasingly efficient as anti-infective agents with increasing degree of isomeric purity. In other word it has surprisingly been shown that while both the agents of the general formula (IIIa) (*cis*-isomers) and the agents of the general formula (IIIb) (*trans*-isomers) display potent anti-infective properties, the isomeric mixtures of the agents of the general formula (IIIa) and (IIIb) show a reduced anti-infective activity. This surprising effect may be seen e.g. in examples 6, 7 and 8.

Particularly, it has surprisingly been shown, during the course of the experiments leading to the present invention, that presence of the trans-isomer inhibit the anti-infective properties of the cis-isomer and that presence of the cis-isomer inhibit the anti-infective properties of the trans-isomer. Even small amounts of isomeric impurity of one isomer 5 may inhibit the anti-infective properties of the other relevant anti-infective isomer.

Consequently, it is generally preferred that the compounds of the general formula (III) are used as pure or substantially pure isomers. Accordingly, the compounds according to this embodiment are preferably used in an isomeric purity of at least 60% such as at 0 least 70%, such as at least 80%, such as at least 90% or even at least 95%, or even at least 98%.

Specific examples of the above-mentioned thioxanthenes include N-dealkyl-*trans-*flupenthixol (or trans-1-(3-(2-fluor-thioxanthen-9-ylidene)propyl)piperazine), N-dealkyl-5 *cis*-flupenthixol (or cis-1-(3-(2-fluor-thioxanthen-9-ylidene)propyl)piperazine), N-deatkyl-*trans*-clopenthixol (or trans-1-(3-(2-chloro-thioxanthen-9-ylidene)propyl)piperazine), N-dealkyl-*cis*-clopenthixol (or cis-1-(3-(2-chlorothioxanthen-9-ylidene)propyl)piperazine), *trans*-4-[3-(2-(trifluoromethyl)thioxanthen-9-ylidene)propyl] 1-methylpiperazine, *cis*-4-3-(2-(trifluoromethyl)thioxanthen-9-ylidene)propyl] 1- methylpiperazine, *trans*-4-[3-(2-chlorothioxanthen-9-ylidene)propyl]1-methylpiperazine, *cis*-4-[3-(2-chlorothioxanthen-9-ylidene)propyl]1-methylpiperazine.

It has been shown during the course of the experiments leading to the present invention that the trans-forms of the compounds according to the invention are the most potent anti-infective agents. Further, the apparent lack of anti-psychotic activity or extrapyramidal side effects of the *trans*-forms makes them particularly attractive for use as anti-infective agents. Accordingly, it is generally preferred that the compounds of the general formula (III) have the *trans* configuration, i.e. the structure shown in the general formula (IIIb).

Thus in a particularly preferred embodiment of the invention n is 2, and each X is hydrogen and R₁₂ is hydrogen or CH₃, the agents of the general formula (IIIb) show a potent anti-infective activity at clinically relevant concentrations. Thus, in a preferred embodiment of the invention, W together with the functional group attached thereto form an alkenyl chain (C=C-(CHX)ₙ-) with an optionally substituted piperazinyl group in the trans configuration. The piperazinyl group is preferably unsubstituted or substituted in the para position (R₁₂). Thus, in a preferred embodiment W together with the functional group attached thereto is CCH-trans-(CH₂)₂-4-methyl-piperazinyl, CCH-trans-CH₂-CH(CH₃)-4-methyl-piperazinyl, CCH-trans-(CH₂)₂-piperazinyl or CCH-trans-CH₂-CH(CH₃)-piperazinyl. In particular, the structure where W together with the functional group attached thereto is CCH-trans-(CH₂)₂-4-piperazinyl is preferred.

Particularly preferred anti-infective agents for the use according to the invention are N-dealkyl-*trans*-flupenthixol and N-dealkyl-*trans*-clopenthixol. Most preferred is N-dealkyl-*trans*-clopenthixol (or trans-1-(3-(2-chloro-thioxanthen-9-ylidene)propyl)piperazine).

As is evident from the formulae shown herein and the definitions associated therewith, certain of the anti-infective agents described herein are chiral. Moreover, the presence of certain unsaturated or cyclic fragments or multiple stereogenic atoms provides for the existence of diastereomeric forms of some of the anti-infective agents. The invention is intended to include all stereoisomers, including optical isomers, and mixtures thereof, as well as pure, partially enriched, or, where relevant, racemic forms. In particular, many of the anti-infective agents described herein may be in the form of *E-* or *Z-*stereoisomers, or mixtures of such isomers.

It should furthermore be understood that the anti-infective agents described herein include possible salts thereof, of which pharmaceutically acceptable salts are of course especially relevant for the therapeutic applications. Salts include acid addition salts and basic salts. Examples of acid addition salts are hydrochloride salts, fumarate, oxalate, etc. Examples of basic salts are salts where the (remaining) counter ion is selected from alkali metals, such as sodium and potassium, alkaline earth metals, such as calcium salts, potassium salts, and ammonium ions (⁺N(R')₄, where the R's independently designate optionally substituted C₁₋₆-alkyl, optionally substituted C₂₋₆-alkenyl, optionally substituted aryl, or optionally substituted heteroaryl). Pharmaceutically acceptable salts are, e.g., those described in Remington's - The Science and Practice of Pharmacy, 20th Ed. Alfonso R.Gennaro (Ed.), Lippincott, Williams & Wilkins; ISBN: 0683306472, 2000, and in Encyclopedia of Pharmaceutical Technology.

The effect of the anti-infective agents may be assayed as described herein and the efficiency of the anti-infective agent against selected microorganisms may be expresses as the MIC value.

The Minimal Inhibitory Concentration, (MIC) is defined as the lowest inhibitory concentration showing no visible growth according to the NCCLS Guidelines.

The anti-infectivity of the anti infective agents described herein, may be assessed by any of the methods available to those skilled in the art, including the in *vitro* assays described in the examples herein. In a preferred embodiment of the invention, the anti-infective agent and the infectious agent (and hence the infectious disease to be treated) exhibit a MIC value of equal to or less than 20 µg/ml when determined as described in the examples herein. More preferably the anti-infective agent and the infectious agent exhibit a MIC value of equal to or less than 16 µg/ml when determined as described in the examples herein. Even more preferably, the MIC value is equal to or less than 8 µg/ml, such as equal to or less than 4 µg/ml, e.g. at the most 4.0. Even more preferably, the MIC value is equal to or less than 2 µg/ml, such as at the most 2.0, at the most 1.0 or even at the most 0.5.

### Therapy, Pharmaceutical compositions and dosages

As explained above, the anti-infective agents described herein are useful for treatment of infectious diseases. Thus, the anti-infective agents described herein may be used for the manufacture of a medicament for the treatment of an infectious disease caused by an infectious agent selected among bacteria, fungi and vira, wherein the anti-infective agents are the sole anti-infective agent.

Thus, in one embodiment the invention relate to the anti-infective agents described herein for use in treatment of an infectious disease caused by an infectious agent selected among bacteria, fungi and vira, wherein the anti-infective agents are the sole anti-infective agent.

In addition, the anti-infective agents described herein are useful for prophylactic treatment of infectious diseases. This may be particularly relevant in situations where a person has a high risk of getting infections, such as immunosuppressed patients or patients undergoing surgery. Thus, the anti-infective agents described herein may also be used for the manufacture of a medicament for the prophylactic treatment of an infectious disease caused by an infectious agent selected among bacteria, fungi and vira, wherein the anti-infective agents are the sole anti-infective agent.

Thus, in another embodiment the invention relate to the anti-infective agents described herein for use in prophylactic treatment of an infectious disease caused by an infectious agent selected among bacteria, fungi and vira, wherein the anti-infective agents are the sole anti-infective agent.

In a further aspect, the present invention is directed to the anti-infective agents described herein for use as medicaments for treatment of multidrug resistant infections.

### Therapy

As will be understood from the disclosure herein, the infectious disease to be treated is normally caused by an infectious agent, such as a bacterium, a virus, or a fungi, in particular a bacterium. The infectious agent is typically naturally-occurring, i.e. a naturally-occurring bacterium, a naturally occurring virus, or a naturally occurring fungi, in particular a naturally-occurring bacterium.

More particularly, the infectious agent may be Gram negative or Gram positive bacteria.

Specific examples include Gram negative bacteria of a genus selected from the group consisting of Escherichia, Proteus, Salmonella, Klebsiella, Providencia, Enterobacter, Burkholderia, Pseudomonas, Acinetobacter, Aeromonas, Haemophilus, Yersinia, Neisseria, Erwinia, Rhodopseudomonas and Burkholderia.

Specific examples of Gram positive bacteria include bacteria from a genus selected from the group consisting of Lactobacillus, Azorhizobium, Streptococcus, Pediococcus, Photobacterium, Bacillus, Enterococcus, Staphylococcus, Clostridium, Butyrivibrio, Sphingomonas, Rhodococcus and Streptomyces.

In other embodiments, the infectious agent is, e.g., from a genus selected from the group consisting of Methanobacierium, Sulfolobus, Archaeoglobu, Rhodobacter and Sinorhizobium.

In still other embodiments, the infectious agent is fungi, such as from the genus Mucor or Candida, e.g., Mucor racemosus or Candida albicans; from genus Crytococcus e.g., Cr. Neoformans; or from Genus Aspergillus, e.g., A. fumingatus.

Toxicity and therapeutic efficacy of the anti-infective agents described herein can be determined by standard pharmaceutical procedures in cell cultures or experimental animals, e.g., by determining the LD₅₀ (the dose lethal for 50% of the population) and the ED₅₀ (the dose therapeutically effective in 50% of the population). The dose ratio between toxic and therapeutic effects is the therapeutic index and it can be expressed as the ratio between LD₅₀ and ED₅₀ (LD₅₀/ED₅₀). Anti-infective agents which exhibit large therapeutic indices are preferred. The data obtained from these cell culture assays or animal studies can be used in formulating a range of dosage for use in human subjects. The dosage of such anti-infective agents lies preferably within a range of circulating concentrations that include the ED₅₀ with little or no toxicity. The dosage may vary within this range depending upon the dosage form employed and the route of administration utilised.

### Pharmaceutical compositions

The anti-infective agents described herein are typically formulated in a Pharmaceutical composition prior to use as a drug substance.

Accordingly, in a further aspect the present invention relates to a pharmaceutical composition comprising an anti-infective agent as described herein and at least one pharmaceutically acceptable carrier or exipient wherein said composition does not comprise further anti-infective agents.

The administration route of the anti-infective agents described herein may be any suitable route that leads to a concentration in the blood or tissue corresponding to a clinically relevant concentration. Thus, e.g., the following administration routes may be applicable although the invention is not limited thereto: the oral route, the parenteral route, the cutaneous route, the nasal route, the rectal route, the vaginal route and the ocular route. It should be clear to a person skilled in the art that the administration route is dependant on the particular anti-infective agent in question, particularly, the choice of administration route depends on the physico-chemical properties of the anti-infective agent together with the age and weight of the patient and on the particular disease or condition and the severity of the same. In general, however, the oral and the parental routes are preferred.

The anti-infective agents described herein may be contained in any appropriate amount in the pharmaceutical composition, and are generally contained in an amount of about 0.1-95% by weight of the total weight of the composition. The composition may be presented in a dosage form, such as a unit dosage form, which is suitable for the oral, parenteral, rectal, cutaneous, nasal, vaginal and/or ocular administration route. Thus, the composition may be in form of, e.g., tablets, capsules, pills, powders, granulates, suspensions, emulsions, solutions, gels including hydrogels, pastes, ointments, creams, plasters, drenches, delivery devices, suppositories, enemas, injectables, implants, sprays, aerosols and in other suitable form.

The pharmaceutical compositions may be formulated according to conventional pharmaceutical practice, see, e.g., "Remington's Pharmaceutical Sciences" and "Encyclopedia of Pharmaceutical Technology", edited by Swarbrick, J. & J. C. Boylan, Marcel Decker, Inc., New York, 1988. Typically, the anti-infective agents described herein are formulated with (at least) a pharmaceutically acceptable carrier or exipient. Pharmaceutically acceptable carriers or exipients are those known by the person skilled in the art.

Pharmaceutical compositions for oral use include tablets which contain an anti-infective agent as described herein, optionally in combination with at least one further anti-infective agent, in admixture with non-toxic pharmaceutically acceptable excipients. These excipients may be, for example, inert diluents or fillers, such as sucrose, sorbitol, sugar, mannitol, microcrystalline cellulose, starches including potato starch, calcium carbonate, sodium chloride, lactose, calcium phosphate, calcium sulfate or sodium phosphate; granulating and disintegrating agents, for example, cellulose derivatives including microcrystalline cellulose, starches including potato starch, croscarmellose sodium, alginates or alginic acid; binding agents, for example, sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone or polyethylene glycol; and lubricating agents, including glidants and antiadhesives, for example, magnesium stearate, zinc stearate, stearic acid, silicas, hydrogenated vegetable oils or talc.

Other pharmaceutically acceptable excipients can be colorants, flavouring agents, plasticisers, humectants, buffering agents, etc.

The tablets may be uncoated or they may be coated by known techniques, optionally to delay disintegration and absorption in the gastrointestinal tract and thereby providing a sustained action over a longer period. The coating may be adapted to release the anti-infective agent in a predetermined pattern, e.g., in order to achieve a controlled release formulation (see below) or it may be adapted not to release the active drug substance until after passage of the stomach (enteric coating). The coating may be a sugar coating, a film coating (e.g. based on hydroxypropyl methylcellulose, methylcellulose, methyl hydroxyethylcellulose, hydroxypropylcellulose, carboxymethylcellulose, acrylate copolymers (Eudragit E®), polyethylene glycols and/or polyvinylpyrrolidone) or an enteric coating (e.g. based on methacrylic acid copolymer (Eudragit® L and S), cellulose acetate phthalate, hydroxypropyl methylcellulose phthalate, hydroxypropyl methylcellulose acetate succinate, polyvinyl acetate phthalate, shellac and/or ethylcellulose).

Furthermore, a time delay material such as, e.g., glyceryl monostearate or glyceryl distearate may be employed.

In addition, the solid tablet compositions as mentioned above may be provided with a coating adapted to protect the composition from unwanted chemical changes, e.g. chemical degradation, prior to the release of the anti-infective agent.

The coating may be applied on the solid dosage form in a similar manner as that described in "Aqueous film coating" by James A. Seitz in "Encyclopedia of Pharmaceutical Technology", Vol 1, pp.337-349 edited by Swarbrick, J. & J. C. Boylan, Marcel Dekker, Inc., New York, 1988.

Formulations for oral use may also be presented as chewing tablets, or as hard gelatin capsules wherein the active ingredient is mixed with an inert solid diluent, for example, potato starch, lactose, microcrystalline cellulose, calcium carbonate, calcium phosphate or kaolin, or as soft gelatin capsules wherein the active ingredient is mixed with water or an oil medium, for example, peanut oil, liquid paraffin, or olive oil.

Powders and granulates may be prepared using the ingredients mentioned above under tablets and capsules in a conventional manner using, e.g., a mixer, a fluid bed apparatus or a spray drying equipment.

Controlled release compositions for oral use may, e.g., be constructed to release the active drug substance by controlling the dissolution and/or the diffusion of the active drug substance.

Dissolution or diffusion controlled release can be achieved by appropriate coating of a tablet, capsule, pellet or granulate formulation of the anti-infective agent, or by incorporating the anti-infective agent in question in, e.g., an appropriate matrix.

A controlled release coating may comprise one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3-butylene glycol, ethylene glycol methacrylate and/or polyethylene glycols.

In a controlled release matrix formulation of the anti-infective agent, the matrix material may comprise, e.g., hydrated metylcellulose, carnauba wax and stearyl alcohol, carbopol 934, silicone, glyceryl tristearate, methyl acrylate-methyl methacrylate, polyvinyl chloride, polyethylene and/or halogenated fluorocarbon.

A controlled release composition of the anti-infective agents described herein, may also be in the form of a buoyant tablet or capsule, i.e. a tablet or capsule which upon oral administration floats on top of the gastric content for a certain period of time. A buoyant tablet formulation of the anti-infective agent in question can be prepared by granulating a mixture of the anti-infective agent, excipients and 20-75% w/w of hydrocolloids, such as hydroxyethylcellulose, hydroxypropylcellulose and hydroxypropylmethylcellulose. The obtained granules can then be compressed into tablets. On contact with the gastric juice, the tablet can form a substantially water-impermeable gel barrier around its surface. This gel barrier takes part in maintaining a density of less than one, thereby allowing the tablet to remain buoyant in the gastric juice.

Powders, dispersible powders or granules suitable for preparation of an aqueous suspension by addition of water are also convenient dosage forms. Formulation as a suspension provides the anti-infective agent in admixture with a dispersing or wetting agent, suspending agent and one or more preservatives.

Suitable dispersing or wetting agents are, for example, naturally-occurring phosphatides, as e.g. lecithin, or condensation products of ethylene oxide with e.g. a fatty acid, a long chain aliphatic alcohol or a partial ester derived from fatty acids and a hexitol or a hexitol anhydrides, for example, polyoxyethylene stearate, polyoxyethylene sorbitol monooleate, polyoxyethylene sorbitan monooleate ,etc.

Suitable suspending agents are, for example, sodium carboxymethylcellulose, methylcellulose, sodium alginate, etc.

The pharmaceutical composition may also be administered parenterally by injection, infusion or implantation (intravenous, intramuscular, intraarticular, subcutaneous or the like) in dosage forms, formulations or e.g. suitable delivery devices or implants containing conventional, non-toxic pharmaceutically acceptable carriers and adjuvants.

The formulation and preparation of such compositions is well-known to those skilled in the art of pharmaceutical formulation. Specific formulations can be found in the textbook entitled "Remington's Pharmaceutical Sciences".

Compositions for parenteral use may be presented in unit dosage forms, e.g. in ampoules, or in vials containing several doses and in which a suitable preservative may be added (see below). The composition may be in form of a solution, a suspension, an emulsion, an infusion device or a delivery device for implantation or it may be presented as a dry powder to be reconstituted with water or another suitable vehicle before use. Apart from the anti-infective agents described herein, the compositions may comprise suitable parenterally acceptable carriers and/or excipients or the active drug substance may be incorporated into microspheres, microcapsules, nanoparticles, liposomes or the like for controlled release. Furthermore, the composition may, in addition, conveniently comprise suspending, solubilising, stabilising, pH-adjusting agents and/or dispersing agents.

In another interesting embodiment of the invention, the pharmaceutical composition is a solid dosage form, such as a tablet, prepared from the particulate material described in WO 03/004001 and WO 2004/062643.

As indicated above, the pharmaceutical compositions may contain the anti-infective agent in the form of a sterile injection. To prepare such a composition, the anti-infective agent is dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution and isotonic sodium chloride solution. The aqueous formulation may also contain one or more preservatives, for example, methyl, ethyl or n-propyl p-hydroxybenzoate. In cases where anti-infective agent is only sparingly or slightly soluble in water, a dissolution enhancing or solubilising agent can be added or the solvent may apart from water comprise 10-60% w/w of propylene glycol or the like.

### Dosages

As discussed in detail previously, an important aspect of the present invention is the realisation that the anti-infective agents described herein are capable of killing infective agents when administered in clinical relevant amounts, i.e. in amounts sufficiently small to avoid the severe side effects normally associated with the anti-infective agents described herein.

It will be understood that the dosage to be administered will be dependent on the administration form (see below). Independently, of the administration form, the anti-infective agent should be administered in clinically relevant amounts, i.e. in amounts which on the one hand exert the relevant therapeutic effect, but on the other hand does not provide severe side effects.

Preferably, an anti-infective agent as described herein is administered in a clinically relevant amount giving rise to a steady state serum concentration of less than 20 mg mg/l. More preferably, the anti-infective agent is administered in a relevant amount giving rise to a steady state serum concentration of less than 10 mg/l such as less than 8.0 mg/l. More preferably, the anti-infective agent is administered in a clinically relevant amount giving rise to a steady state serum concentration of less than 7.0 mg/l, such as less than 6.0 mg/l, e.g. less than 8.0 mg/l. Even more preferably, the anti-infective agent is administered in a clinically relevant amount giving rise to a steady state serum concentration of less than 4.0 mg/l, such as less than 3.0 mg/l, e.g. less than 2.0 mg/l. Most preferably, the anti-infective agent is administered in a clinically relevant amount giving rise to a steady state serum concentration of less than 1.5 mg/l, e.g. about 1.0 mg/l or about 0.5 mg/l

In other words, the anti-infective agent is preferably administered in a clinically relevant amount giving rise to a steady state serum concentration in the interval of from 0.01 ug/l to less than 20.0 mg/l such as 0.01 µg/l to less than 10.0 mg/l and such as 0.01 µg/l to less than 8.0 mg/l, such as in the interval of from 0.02 µg/l to 7.0 mg/l, e.g. in the interval of from 0.04 µg/l to 6.0 mg/l. More preferably, the steady state serum concentration of the anti-infective agent is in the interval of from 0.06 µg/l to 5.0 mg/l, such as is in the interval of from 0.08 µg/l to 4.0 mg/l, e.g. in the interval of from 0.1 µg/l to 3.0 mg/l. Even more preferably, the steady state serum concentration of the anti-infective agent is in the interval of from 0.2 µg/l to 2.0 mg/l, such as in the interval of from 0.4 µg/l to 2.0 mg/l, e.g. in the interval of from 0.5 µg/l to 2.0 mg/l. Still more preferably, the steady state serum concentration of the anti-infective agent is in the interval of from 0.6 µg/l to 2.0 mg/l, such as in the interval of from 0.8 µg/l to 2.0 mg/l_{,} e.g. in the interval of from 0.9 µg/l to 2.0 mg/l. Most preferably, the steady state serum concentration of the anti-infective agent is in the interval of from 1.0 µg/l to 2.0 mg/l, such as in the interval of from 1.5 µg/l to 2.0 mg/l, e.g. in the interval of from 1.5 µg/l to 1.5 mg/l.

The anti-infective agent is preferably administered in an amount of about 0.1 to 3000 mg per day, such as about 0.5 to 2000 mg per day. As will be understood by the skilled person, the actual amount to be administered will *inter alia* be dependent on the administration route, i.e. whether the anti-infective agent is administered orally, intravenous, intramuscular, etc.

For compositions adapted for oral administration for systemic use, the dosage is normally 1 mg to 3 g per dose administered 1-4 times daily for 1 day to 12 months depending on the infectious disease to be treated.

For parenteral administration, in particular intravenous administration, a dose of about 0.1 to about 2000 mg per day is convenient. For intravenous administration a dose of about 0.1 to about 2000 mg per day administered for 1 day to 12 months is convenient.

The above-mentioned steady state serum concentrations and dosages will give rise to the desired clinical effects and, at the same time, avoid the severe side effects normally associated with the anti-infective agents described herein. Some of the anti-infective agents described herein, in particular the anti-infective agents of the general formula IIIb, may however be administered in higher amounts, thereby giving rise to steady state serum concentrations above the levels indicated above. This is due to the fact that these anti-infective agents are expected not to exhibit severe side effects, even when administered in higher amounts.

The invention is further illustrated by the below, non-limiting, examples.

### MATERIALS AND METHODS

### Bacteria

Clinical isolates were obtained from USA, Canada, Europe and Middle East, and standard control strains were obtained from ATCC (American Type Culture Selection USA) and CCUG (Control Culture University of Göteborg, Sweden). The collection included multi resistant isolates and represents clinical important bacteria and fungi.

The resistant cells were approximately 10 to 1000 times more resistant compared to sensitive cell lines and maintained a stable drug resistance phenotype when grown in drug-free medium. All Staphylococci were typed in order to ensure that the isolates did not represent the same clone/strain.

### Drugs

Drugs were dissolved in small amounts of water or 1% DMSO (final culture concentration of DMSO less than 0.05% DMSO) before dilution with medium. Solutions were freshly prepared for each experiment. Purity of compounds were > 95%.

### Effect of drugs on microbial cell growth

Cell growth was tested using the Minimal Inhibitory Concentration (MIC) susceptibility tests by use of the microdilution broth method in accordance to the NCCLS Guidelines (NCCLS Guidelines, Methods for Dilution Antimicrobial Susceptibility Tests for Bacteria That Grow Aerobically; Approved Standard, Sixth Edition, Volume 23; Number 2). The minimum inhibitory concentration (MIC), is defined ad the lowest concentration of drug which inhibits growth of the test organism, in the sense that no visible growth is detected (total inhibition of growth). In example 2 MIC of the compounds used on fungal microorgansims was determined from the IC90 measurements according to NCCLS Guidelines.

A log phase culture of bacteria was diluted with fresh pre-warmed Mueller-Hinton medium and adjusted to a defined OD at 600 nm in order to give a final concentration of 1 x 10⁴⁻⁵ bacteria/ml medium. The bacterial culture was transferred to microtiter-plates and culture was added to each well. Drug was added to the bacterial culture in the wells as two-fold dilution series of drug in order to give final concentrations ranging from 0.03 to 128 µg/ml. Trays were incubated at 37°C by shaking in a robot analyzer, PowerWaveₓ, software KC^{4,} Kebo.Lab, Copenhagen, for 16 h and optical densities were measured at 600 nm during the incubation time in order to record growth curves. Wells containing bacterial culture without drug were used as controls to ensure correct inoculum size and bacterial growth during the incubation. Cultures were tested in order to detect contaminations. Each experiment was repeated in triplicate. MIC values represent the mean values of two separate triplicate experiments. Intra-and interassay variation was < 5%.

### Definition of growth inhibitory effect of anti-infective agents

The bacterial growth in the wells is described by the lagphase i.e. the period until (before) growth starts, the logphase i.e. the period with maximal growth rate, the steady-statephase followed by the deathphase. These parameters are used when evaluating the inhibitory effect of the drug on the bacterial growth, by comparing growth curves with and without drug.

Total inhibition of bacterial growth is defined as: OD (16h) = OD (0h) or no visible growth according to NCCLS Guidelines.

Inhibition 90 (IC90) is defined as: OD responding a 90% growth inhibition.

### EXAMPLES

### Example 1: Effect of dealkylated or demethylated phenothiazines comparative and thioxanthenes on a clinically relevant isolate.

A clinically relevant isolate of Staphylococcus epidermidis was cultured and assayed as described above for susceptibility towards the compounds listed in table 1. The results are shown in Table 1.

**Table 1. Effect of dealkylated or demethylated phenothiazines and thioxanthenes on a multiresistant clinical isolate of Staphylococcus epidermidis.**

| **Compound** | **Group** | **MIC ug/ml** |
|---|---|---|
| N-desmethyl-chlorpromazinePhenothiazine | | 16 |
| N-deshydroxy-ethyl-Fluphenazine (Phe nothiazine) | | 16 |
| Desmethyl-perphenazin (Phe nothiazine) | | 16 |
| N-desmethyl- Chlorprothixen (Phe nothiazine) | | 16 |
| N-dealk-trans-clopenthixol (Thixoan thence) | | 0,5 |
| N-dealkyl-trans-flupenthixol (Thioxan thene) | | 1.0 |
| N-dealkyl-cis-flupenthixol (Thioxan thene) | | 16 |
| N-dealkyl-cis-clopenthixol (Thioxan thence) | | 16 |

### Example 2. Antibacterial effect of Demethylated/dealkylated phenothiazine (comparative) or thioxanthene compounds on clinical isolates of fungi.

The antibacterial effect of demethylated/dealkylated phenothiazine or thiaxanthene compounds were studied by growth inhibition studies exposing cells to 0-32 µg/ml of drug. Each experiment was repeated in triplicate. MIC values represent the mean values of two separate triplicate experiments.

4 clinical isolates of Candida sp. (including 3 fluconazole resistant isolates) were subcultures for 24 h on Sabouraud glucose agar before susceptibility testing. Broth microdilution tests were performed according to NCCLS document M27-A (Ref: National Commitee for Clinical Laboratory Standards. (1997). *Reference Method for Broth Dilution Antifungal Susceptibility Testing of Yeasts: Approved Standard M27-A.* NCCLS, Wayne, PA.). Microtitre plates were read spectrophotometrically at 530 nm, after mixing the wells by pipetting to resuspend yeast sediments. In this experiment, the MIC was defined as the lowest drug dilution resulting in 90% growth inhibition. Results are shown in Table 2 below.

**Table 2. Antibacterial effect on clinical isolates of fungi.**

| **Strain** | **MIC µg/ml N-dealk-trans clopenthixol** | **MIC ug/ml N-dealkyl-trans flupenthixol** | **MIC ug/ml N-desmethyl-Chlorprothixen Phenothiazine** | **MIC ug/ml Desmethyl perphenazine** |
|---|---|---|---|---|
| 1. Candida albicants | 1 | 0.5 | 16 | 16 |
| FR* | 1 | 1 | 16 | 16 |
| 2. Candida glabrata | 0.5 | 1 | 16 | 16 |
| FR* | | | | |
| 4. Candida glabrata FR* | 0.5 | 0.5 | 16 | 16 |
| 5. Candida glabrata | | | | |
| FR: Fluconazole resistant | | | | |

The results show that the thioxanthene compounds tested exhibit strong antifungal activity against the clinical isolates of Candida spincluding all of the multi resistant isolates. Some inhibitory activity of the phenothiazine compounds was shown, however, the effect was inferior when compared to the effect of the thioxanthene compounds.

### Examples 3: Antibacterial effect of N-dealkyl-clopenthixol against a broad spectrum of bacterial species.

A broad spectrum of bacteria were cultured and assayed as described above for susceptibility towards N-dealkyl-clopenthixol. The results are shown in Table 3 below.

**Table 3a. Antibacterial effect of N-dealkyl-clopenthixol**

| **Microorganism** | **No of strains** | **No of re sistant strains** | **Trans-compound MIC ug/ml (mean) range** | **Trans-com pound IC90 ug/ml (mean)** | **Cis-compound MIC ug/ml (mean)** |
|---|---|---|---|---|---|
| Staphylococci, Micrococci. Including MRSA | 30 | 20 | | | |
| | | (20 MRSA) | 1 | 0.5 | 16 |
| | | | 0.5-2 | | |
| Streptococci | 30 | 20 | 1 | 0.5 | 16 |
| | | | 0.5-2 | | |
| Gram negative sp. | 25 | 20 | 2 | 2 | >16 |
| | | | 0.5-2 | | |

**Table 3b. Antibacterial effect of N-dealkyl-trans flupenthixol**

| Microorganism | No of strains | No of resistant strains | Trans-compound MIC ug/ml (median) |
|---|---|---|---|
| Staphylococci, Micrococci. Including MRSA | 30 | 20 | |
| | | (20 MRSA) | 1,5 |
| Streptococci | 30 | 20 | 1,5 |
| Gram negative sp. | 25 | 20 | 2 |

As seen, N-dealkyl-trans flupenthixol exhibits a potent antimicrobial effect against multiresistant bacterial isolates.

The results in Table 3a and 3b show that the tested compounds N-dealkyl-clopenthixol and N-dealkyl-trans flupenthixol exhibit strong antimicrobial activity against all of the 60 gram positive clinical isolates including all of the multi resistant isolates. Further it is demonstrated that the compounds also exhibit strong antimicrobial activity against all of the 25 gram negative clinical isolates including the resistant isolates, Interestingly, the superiority of N-dealkyl-trans-clopenthixol when compared to the cis-compound N-dealkyt-cis-clopenthixol is demonstrated.

No difference in susceptibility towards N-dealkyl-trans-clopenthixol and N-dealkyl-trans flupenthixol was seen observed between the resistant and susceptible isolates.

### Example 4: Antimicrobial/antifungal effect of the trans-forms and cis-forms of the compounds according to the invention on multi resistant bacterial and fungi.

Cultured microorganisms were assayed as described above. Cis- and trans-isomers of N-dealkyl-clopenthixol was added to the cultures. Results are shown in table 4 below.

**Table 4. The antimicrobial effect of cis-forms and trans-forms.**

| **Microorganism** | **MIC trans-compound ug/ml** | **MIC cis-compound ug/ml** |
|---|---|---|
| Enterococcus faecium multiresistant isolate | 0.5 | 16 |
| Candida glabrata Multiresistant isolate | 1.0 | > 16 |

The results in Table 4 show that the cis-isomer is the weaker antimicrobial agent when compared to the trans-isomer. In this experiment, the effect of the pure trans-isomer was 32 times higher than the effect of the cis-isomer.

### Example 5. Effect of N-dealkyl-trans-clopenthixol on resistant clinical isolates of Enterococcus faecalis and Enterococcus faecium.

Clinically relevant isolates were cultured and assayed as described above. The results are shown in Table 5.

**Table 5. Effect of N-dealkyl-trans-clopenthixol on resistant clinical isolates**

| **Strain** | **Resistance** | **Trans-compound MIC ug/ml** | **IC90 Trans-compound** | **Cis-compound MIC ug/ml** |
|---|---|---|---|---|
| Enterococcus faecalis 1 | VanA | 1 | 0.6 | >8 |
| Enterococcus faecalis 2 | VanA | 1 | 0.6 | >8 |
| Enterococcus faecalis 3 | VanA | 1 | 0.06 | >8 |
| Enterococcus faecalis 4 | VanB | 1 | 0.6 | >8 |
| Enterococcus faecalis 5 | Van B | 2 | 1 | >8 |
| Enterococcus faecalis 6 | VanB | 1 | 0.6 | >8 |
| Enterococcus faecalis 7 | VanA | 1 | 0.6 | >8 |
| Enterococcus faecalis 8 | HLAR | 1 | 0.6 | >8 |
| Enterococcus faecalis 9 | HLAR | 1 | 0.6 | >8 |
| Enterococcus faecalis 10 | BLR,CR | 1 | 0.6 | >8 |
| Enterococcus faecium 11 | VanA | 1 | 0.6 | >8 |
| Enterococcus faecium 12 | VanA | 1 | 0.6 | >8 |
| Enterococcus faecium 13 | VanB | 1 | 0.5 | >8 |
| Enterococcus faecium 14 | HLAR | 1 | 0.5 | >8 |
| Enterococcus faecium 15 | HLAR | 1 | 0.5 | >8 |
| Enterococcus faecium 16 | BLR,CR | 1 | 0.6 | >8 |
| Enterococcus faecium 17 | VanB | 1 | 0.5 | >8 |
| Enterococcus faecium 18 | VanB | 1 | 0.6 | >8 |
| Enterococcus faecium 19 | VanB | 1 | 0.6 | >8 |

| | | | | |
|---|---|---|---|---|
| VanB: This isolate exhibits *vanB*-glycopeptideresistance which affects primarily vancomycin and not teicoplanin. VanA: This isolate exhibits *vanA*-glycopeptideresistance which affects both vancomycin and teicoplanin. HLAR: This isolate exhibits high level aminaglycoside resistance. BLR,CR: This isolate exhibits betalactam and carbapenem resistance. | | | | |

The experiment shows that the tested compound N-dealkyl-trans-clopenthixol exhibits strong antimicrobial) activity against resistant and multiresistant isolates including vancomycin resistant, teicoplanin resistant and high level aminoglycoside resistant Enterococcus species. As seen, the antimicrobial power of the trans-form are superior to the cis-form.

### Example 6: Antibacterial effect of the mixture of cis-trans metabolite compounds compared to the effect of cis and trans compounds alone.

In order to test the surprisingly week effect of the cis/trans isomeric mixture of N-dealkyl-clopenthixol previously described in the literature (Kristensen et al.) and in order to compare with the antibacterial effect of the cis and trans-isomers alone, the cis and trans isomers of N-dealkyl-clopenthixol were studied alone and in a 1:1 mixture using the broth microdilution tests, performed according to NCCLS Guidelines, as described above. Results are presented in Table 6.

**Table 6: Effect of the mixture of cis-trans N-dealkyl-clopenthixol compared to the effect of cis-and trans- N-dealkyl-clopenthixol alone.**

| **Microrganism** | **MIC trans-compound ug/ml** | **MIC cis-compound ug/ml** | **MIC mixture 1:1, ug/ml** |
|---|---|---|---|
| Staphylococcus epidermidis mul tiresistant isolate | 0.5 | > 8 | > 4 |

As seen in Table 6 we found a very strong antimicrobial activity of the trans-compound alone, a week effect of the cis-compound alone and, surprisingly, only a week effect of the 1:1 mixture even though it contains the strong trans-compound in a concentration far above the concentration needed for 100% inhibition of bacterial growth when tested alone (the observed MIC of the mixture was > 2 ug/ml trans compound + 2 ug/ml cis compound, compared to 0.5 ug/ml trans compound when tested alone). This surprising finding indicates that the cis compound has an inhibitory effect on the anti-infectivity of the trans-compound. This may be one of the explanations for the weak effect of the isomeric mixture reported by Kristiansen et al.

### Example 7: Inhibition of the antimicrobial effect of the trans-compound by the cis-compound.

In order to verify the findings presented in example 6 that the cis-compounds have an inhibitory effect on the anti-infectivity of the trans-compound a further experiment was performed using another clinically relevant resistant bacterial isolate S. Aureus. The assay was performed as described above and results are shown in table 7 below.

**Table 7. Presence of N-dealkyl-cis clopenthixol has an inhibitory effect on the antimicrobial effect of the corresponding trans-form N-dealkyl-transclopenthixol.**

| **Trans-compound µg/ml** | **Cis-compound µg/ml** | **Growth inhibition** | **Effect index(trans compound)*** |
|---|---|---|---|
| 0,5 | 0 | 100% | 100 |
| 0,5 | 0,5 | No inhibition | 0 |
| 1 | 0,5 | No inhibition | 0 |
| 3 | 0,5 | 100% | 17 |

| | | | |
|---|---|---|---|
| * Index 100 trans-compound: minimal concentration needed for 100% inhibition (MIC) when the trans-compound is used alone = 0,5 ug/ml. If 100% inhibition is not achieved, effect is noted as zero. Effect index trans-compound: MIC(trans alone)/ MIG(trans in trans-cis mixture) x 100 | | | |

The results in Table 7 confirm the surprising finding that the cis-compound inhibits the antimicrobial effect of the trans-compound. In this experiment the antimicrobial effect is present at a concentration of trans compound of 3.0 ug/ml in the presence of 0.5 ug/ml cis compound, whereas the anti-microbial effect is present at 0.5 0 ug/ml in the absence of cis compound. Accordingly the anti-infective activity decreases 83% from 100 to 17.

### Example 8: Inhibition of the antimicrobial effect of the cis-compound by the trans-compound.

In order to test if the trans-compounds have an inhibitory effect on the anti-infectivity of the cis-compound a further experiment was performed using the clinically relevant resistant bacterial isolate S. Aureus. The assay was performed as described above and results are shown in Table 8 below.

**Table 8. Inhibition of the antimicrobial effect of the N-deatkyl -cis clopenthixol by the corresponding trans-form N-dealkyl-transclopenthixol. Bacterial isolate: S. Aureus.**

| **Cis-compound µg/ml** | **Trans-compound µg/ml** | **Growth inhibition** | **Effect index(cis-compound)*** |
|---|---|---|---|
| 16 | 0 | 100% | 100 |
| 16 | 0,25 | No inhibition | 0 |
| 17 | 0,25 | No inhibition | 0 |
| 18 | 0,25 | 100% | 89 |

| | | | |
|---|---|---|---|
| * Index 100 cis-compound: minimal concentration needed for 100% inhibition (MIC) when the cis-compound is used alone = 16 ug/ml. If 100% inhibition is not achieved, effect is noted as zero. Effect index cis-compound: MIC(cis alone)/ MIC(cis in cis-trans mixture) x 100 | | | |

The results in table 8 show the surprising finding that the trans-compound inhibits the antimicrobial effect of the cis-compound. The antimicrobial effect of the cis-compound decreases 11% from 100 to 89 in the presence of minor amounts (less than the MIC values of trans-compound), of the trans-compound.

These findings are highly surprising and suggest that both cis and trans forms of the compounds according to the present invention posses anti-infective properties, whereas the mixture of the two isomers is less anti-infective.

### Example 9. Anti-microbial ffect of N-dealkyl-Trans-clopenthixol in a mouse peritonitis/sepsis model

### Bacteria.

A clinical multiresistant isolate of Enterococcus faecalis BG VSE-92 from human urine was used.

### Animals.

Female NMRI mice (age, approximately 6 to 8 weeks; weight, 30 ± 2 g) were used for the mouse pneumonia peritonitis model (as described below).

Bacterial suspensions were prepared from fresh overnight cultures (made from frozen stock cultures) on 5% blood agar plates as described above. The inoculum for the mouse peritonitis model was prepared immediately before use and was adjusted at 540 nm of giving a density of approximately 10⁷ CFU/ml. The size of the inoculum was determined by viability counting on 5% blood agar.
The mice were injected intraperitoneally with 0.5 ml of the anterococcal suspension, resulting in bacteremia within 1 h of inoculation. Antibiotic therapy was initiated 1 h after inoculation. N-dealkyl- trans-clopenthixol was administered subcutaneously in the neck region in a volute of 0.7 ml per dose. Ten mice were in each treatment group. Inoculated untreated control mice were included in all trials. (Method reference: Erlandsdottir et al; Antimicrob Agents Chemother. 2001 Apr;45(4):1078-85)

**Table 9a. Treatment regimes of infected mice.**

| Groups | Treatment |
|---|---|
| Controls | None |
| N-dealkyl-trans-clopenthixol | 25 mg /kg s.c. |

The effects of the various treatment regimens were determined during 6 h of treatment by evaluation of bacterial counts in the peritoneal fluid. After the mice were killed, blood were immediately diluted 10-fold in saline, from which 20 µl was plated onto 5% blood agar plates in spots, with subsequent counting of colonies after incubation overnight at 35°C. The lowest detection levels for bacterial counts in blood were 50 CFU/ml.
The bactericidal efficacies of the treatment regimens in the mouse models were calculated by subtracting the results for each treated mouse from the mean results for control mice at the end of therapy (6 h). A P value of <0.05 was considered significant. All statistical comparisons were two-tailed.

The bactericidal activity of N-dealkyl- trans-clopenthixol in mouse blood is shown in Table 9b. As seen, when the mice were treated with N-dealkyl- trans-clopenthixol the number of bacteria per ml of blood decreased approx. 2 log (p< 0.05) thereby showing a strong anti-microbial activity of N-dealkyl- trans-clopenthixol in infected mouse.

**Table 9b. Bacteria/ml of blood in treated and non-treated infected mice, 6 hours after inoculation.**

| Mouse no | Treatment | Cfu/ml blood (median) | Log (cfu/ml blood) (median) |
|---|---|---|---|
| 1-10 | none | 7,5 x10⁶ | 6,88 |
| 11-20 | Vehikel (saline) | 9,5 x 10⁵ | 5,98 |
| 21-30 | N-d-tc*, 25 mg/kg s.c | 1.55x 10⁴ | 4,19 |

| | | | |
|---|---|---|---|
| *: N-dealkyl.trans-clopenthixol | | | |

### Example 10 - antimicrobial effects of thioxanthene derivatives on fungals

The antimicrobial effect of N-dealkyl-trans-clopenthixol and N-dealkyl-trans-flupenthixol was studied by exposing cells to 0-8 µg/ml of the compounds in two-fold dilutions. Each experiment was repeated in tripleduplicate. MIC values represent the mean values of two separate experiments.

### Fungal strain:

Clinical isolate of a fluconazole resistant Candida albicans from a patient with candidemia.

The isolates were subcultured for 24 h on Sabouraud glucose agar before susceptibility testing. Broth microdilution tests were performed according to NCCLS document M27-A (Ref: National Commitee for Clinical Laboratory Standards. (1997). *Reference Method for Broth Dilution Antifungal Susceptibility Testing of Yeasts: Approved Standard M27-*A. NCCLS, Wayne, PA.)
Microtitre plates were read spectrophotometrically at 530 nm, after mixing the wells by pipetting to resuspend yeast sediments. The MIC was defined as the lowest drug dilution resulting in 80% growth inhibition.

**Table 10**

| Fungal Strain | MIC µg/ml Fluconazole (FL) | MIC µg/ml N-dealkyl-Transclopenthixol | MIC µg/ml N-dealkyl-Transflupenthixol |
|---|---|---|---|
| Candida albicans | 128 | 3 | 3 |

As seen, N-dealkyl-trans-flupenthixol and N-dealkyl-trans-clopenthixol exhibits a potent antifungal effect.

### Example 11 - Enhancing effects of thioxanthene derivatives on anti-viral compounds

The antiviral effect of N-dealkyl-trans-flupenthixol and N-dealkyl-trans-clopenthixol was studied by checkerboard studies exposing HIV infected cells to 0-3 µM of *trans-*clopenthixol. Each experiment was repeated in tripleduplicate. MIC values represent the mean values of two separate experiments.

### Methods:

### Viruses and cells.

The HIV-1 strain HTLV-IIIB were propagated in H9 cells at 37°C, 5% CO2 using RPMI 1640 with 10% heat-inactivated foetal calf serum (FCS) and antibiotics (growth medium). Culture supernatant was filtered (0.45 nm), aliquotted, and stored at -80°C until use. The HIV-1 strain was obtained from NIH AIDS Research and Reference Program.

### Compounds.

N-dealkyl-trans-flupenthixol and N-dealkyl-trans-clopenthixol.

### Inhibition of HIV-1 replication.

Compounds were examined for possible antiviral activity against strain IIIB of HIV-1 using MT4 cells as target cells. MT4 cells were incubated with virus (0.005 MOI) and growth medium containing the test dilutions of compound(s) for six days in parallel with virus-infected and uninfected control cultures without compound added. Expression of HIV in the cultures was indirectly quantified using the MTT assay as previously described. Compounds mediating less than 30% reduction of HIV expression were considered without biological activity. Compounds were tested in parallel for cytotoxic effect in uninfected MT4 cultures containing the test dilutions of compound as described above. Cultures for test of both antiviral activity and cytotoxic effect were set up in tri-pleduplicates, 200 ml per culture in micro titre plates. A 30% inhibition of cell growth relative to control cultures was considered significant. The 50% inhibitory concentration was determined by interpolation from the plots of percent inhibition versus concentration of compound.

EC50 is defined as the effective concentration that inhibits 50% of viral production, 50% of viral infectivity, or 50% of the virus-induced cytopathic effect.
CC50 is defined as the inhibitory concentration that reduces cellular growth or viability of uninfected cells by 50%.

### Results

As seen in Table 11, N-dealkyl-trans-flupenthixol and N-dealkyl-trans-clopenthixol exhibits antiviral effect in vitro and thus may be sufficient to inhibit viral strains in vivo.

**Table 11. Antiviral effect of N-dealkyl-trans-flupenthixol and N-dealkyl-transclopenthixol. Concentrations in µM. (see text).**

| EC50 N-dealkyl-trans clopenthixol | CC50 N-dealkyl-trans clopenthixol | EC50 N-dealkyl-trans flupenthixol | CC50 N-dealkyl-trans flupenthixol |
|---|---|---|---|
| 2 | >3 | 2 | > 3 |

| | | | |
|---|---|---|---|
| EC50 is defined as the effective concentration that inhibits 50% of viral production, 50% of viral infectivity, or 50% of the virus-induced cytopathic effect CC50 is defined as the inhibitory concentration that reduces cellular growth or viability of uninfected cells by 50%. | | | |

Viral test Method Reference: Petersen L, Jørgensen PT, Nielsen C, Hansen TH, Nielsen J, Pedersen EB. Synthesis and Evaluation of Double-Prodrugs against HIV. Conjugation of D4T with 6-Benzyl-1-(ethoxymethyl)-5-isopropyluracil (MKC-442, Emivirine) Type Reverse Transcriptase Inhibitors via the SATE Prodrug Approach. J. Med. Chem. 2005, 48, 1211-1220.

## Claims

1. An anti-infective agent of the general formula (I) 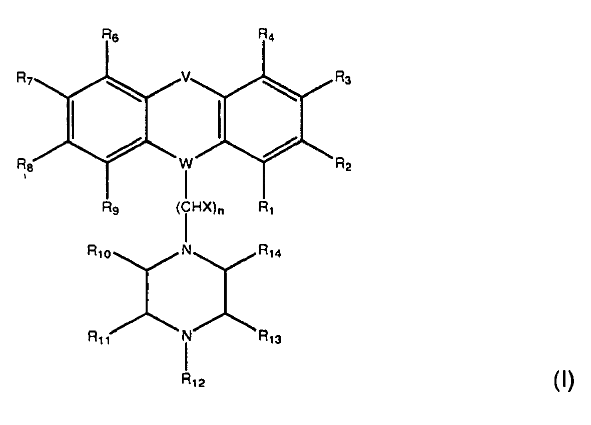 wherein
W is C=CH;
V is selected from the group consisting of S, SO₂, SO, O or NH;
n is an integer in the range of from 1 to 6;
each X is individually selected from the group consisting of hydrogen, halogen, hydroxy, amino, nitro, optionally substituted C₁₋₆-alkyl and optionally substituted C₁₋₆-alkoxy;
R₁, R₂, R₃, R₄, R₅, R₆, R₇, R₈, R₉, R₁₀, R₁₁, R₁₃ and R₁₄ are each individually selected from the group consisting of hydrogen, halogen, hydroxy, amino, nitro, optionally substituted C₁₋₆-alkyl, optionally substituted C₂₋₆-alkenyl, optionally substituted C₂₋₆-alkynyl and optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl optionally substituted C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkyl-carbonylamino, mono- and di(C₁₋₆-alkyl)amino-C₁₋₆-alkyl-carbonylamino, amin-C₁₋₆-alkyl-amino, mono- and di(C₁₋₆-alkyl)amino-C₁₋₆-alkyl-amino, cyano, guanidino, carbamido, C₁₋₆-alkanoyloxy, C₁₋₆-alkylsulphonyl, C₁₋₆-alkylsulphinyl, C₁₋₆-alkylsulphonyloxy, aminosulfonyl, mono- and di(C₁₋₆-alkyl)aminosulfonyl, and optionally substituted C₁₋₆-alkylthio; and
R₁₂ is hydrogen, hydroxy, amino, nitro, halogen, CH₂Y, CHY₂ and CY₃, wherein each Y is individually selected among hydrogen, hydroxy, amino, nitro or halogen;
or a salt thereof for use in the treatment or prophylaxis of an infectious disease, caused by an infectious agent selected among pathogenic bacteria, fungi and vira, wherein the anti-infective agent is used as sole anti-infective agent.

2. Agent for use according to claim 1, wherein R₂ is selected from the group consisting of F, Cl, Br, I, CH₂Y, CHY₂ and CY₃, wherein Y is a halogen atom.

3. Agent for use according to claim 2, wherein R₁, R₂, R₃, R₄, R₅, R₆, R₇, R₈, R₉, R₁₀, R₁₁, R₁₃ and R₁₄ are each individually selected from the group consisting of hydrogen, optionally substituted C₁₋₆-alkyl and optionally substituted C₁₋₆-alkoxy.

4. Agent for use according to any of the preceding claims, wherein V is S or SO.

5. Agent for use according to any of claims 1-4, wherein n is an integer in the range of from 1 to 5.

6. Agent for use according to any of claims 1-5 wherein R₁₂ is selected from the group consisting of hydrogen, CH₃ and CH₂OH.

7. Agent for use according to claim 6, wherein W together with the functional group attached thereto is CCH-(CH₂)₂-4-methyl-piperazinyl, CCH-CH₂-CH(CH₃)-4-methyl-piperazinyl, CCH-(CH₂)₂-piperazinyl or CCH-CH₂-CH(CH₃)-piperazinyl.

8. Agent for use according to any of the preceding claims 1-7, wherein the anti-infective compound has a *trans* configuration.

9. Agent for use according to any of the preceding claims 1-7, wherein the anti-infective compound has a cis configuration.

10. Use of an agent according to any of the preceding claims 1-9 for the manufacture of a medicament for the treatment or prophylaxis of an infectious disease caused by an infectious agent selected among pathogenic bacteria, fungi and vira, wherein the anti-infective agent is used as sole anti-infective agent.

11. Use according claim 10, wherein said agent is used or administered in a clinically relevant amount.

12. Use according to claim 11, wherein said agent is used or administered in a clinically relevant amount giving rise to a steady state serum concentration of less than 20.0 mg/l.

13. Use according to claim 12, wherein said steady state serum concentration is in the interval of from 0.01 µg/l to less than 20.0 mg/l.

14. A pharmaceutical composition comprising an agent as defined in any of claims 1-9 and at least one pharmaceutically acceptable carrier or exipient, wherein said composition does not comprise further anti-infective agents.

15. The pharmaceutical composition according to claim 14, wherein said composition is in a unit dosage form.

## Patentansprüche

1. Anti-Infektionsmittel der allgemeinen Formel (I) 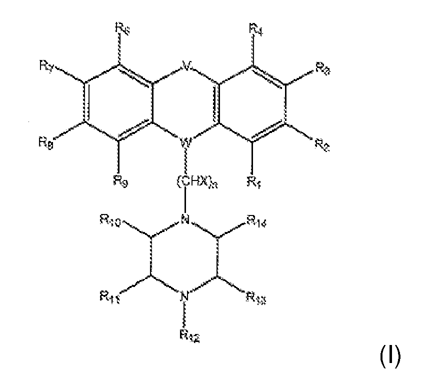 wobei
W C=CH ist;
V ausgewählt ist aus der Gruppe bestehend aus S, SO₂, SO, O oder NH; n eine ganze Zahl im Bereich von 1 bis 6 ist;
jedes X individuell ausgewählt ist aus der Gruppe bestehend aus Wasserstoff, Halogen, Hydroxy, Amino, Nitro, gegebenenfalls substituiertem C₁₋₆-Alkyl und gegebenenfalls substituiertem C₁₋₆-Alkoxy;
R₁, R₂, R₃, R₄, R₅, R₆, R₇, R₈, R₉, R₁₀, R₁₁, R₁₃ und R₁₄ jeweils individuell ausgewählt sind aus der Gruppe bestehend aus Wasserstoff, Halogen, Hydroxy, Amino, Nitro, gegebenenfalls substituiertem C₁₋₆-Alkyl, gegebenenfalls substituiertem C₂₋₆-Alkenyl, gegebenenfalls substituiertem C₂₋₆-Alkinyl und gegebenenfalls substituiertem C₁₋₆-Alkoxy, gegebenenfalls substituiertem C₂₋₆-Alkenyloxy, Carboxy, gegebenenfalls substituiertem C₁₋₆-Alkoxycarbonyl, gegebenenfalls substituiertem C₁₋₆-Alkylcarbonyl, Formyl, gegebenenfalls substituiertem C₁₋₆-Alkylsulfonylamino, gegebenenfalls substituiertem Aryl, gegebenenfalls substituiertem Aryloxycarbonyl, gegebenenfalls substituiertem Aryloxy, gegebenenfalls substituiertem Arylcarbonyl, gegebenenfalls substituiertem Arylamino, Arylsulfonylamino, gegebenenfalls substituiertem Heteroaryl, gegebenenfalls substituiertem Heteroaryloxycarbonyl, gegebenenfalls substituiertem Heteroaryloxy, gegebenenfalls substituiertem Heteroarylcarbonyl, gegebenenfalls substituiertem Heteroarylamino, Heteroarylsulfonylamino, gegebenenfalls substituiertem Heterocyclyl, gegebenenfalls substituiertem Heterocyclyloxycarbonyl, gegebenenfalls substituiertem Heterocyclyloxy, gegebenenfalls substituiertem Heterocyclylcarbonyl, gegebenenfalls substituiertem Heterocyclylamino, Heterocyclylsulfonylamino, Mono- und Di(C₁₋₆-alkyl)amino, Carbamoyl, Mono- und Di(C₁₋₆-alkyl)aminocarbonyl, Amino-C₁₋₆-alkylaminocarbonyl, Mono- und Di(C₁₋₆-alkyl)amino-C₁₋₆-alkyl-aminocarbonyl, C₁₋₆-Alkylcarbonylamino, Amino-C₁₋₆-alkyl-carbonylamino, Mono- und Di(C₁₋₆-alkyl)amino-C₁₋₆-alkyl-carbonylamino, Amino-C₁₋₆-alkyl-amino, Mono- und Di(C₁₋₆-alkyl)amino-C₁₋₆-alkyl-amino, Cyano, Guanidino, Carbamido, C₁₋₆-Alkanoyloxy, C₁₋₆-Alkylsulfonyl, C₁₋₆-Alkylsulfinyl, C₁₋₆-Alkylsulfonyloxy, Aminosulfonyl, Mono- und Di(C₁₋₆-alkyl)aminosulfonyl, und gegebenenfalls substituiertem C₁₋₆-Alkylthio, und
R₁₂ Wasserstoff, Hydroxy, Amino, Nitro, Halogen, CH₂Y, CHY₂ und CY₃ ist, wobei jedes Y individuell ausgewählt ist aus Wasserstoff, Hydroxy, Amino, Nitro oder Halogen;
oder ein Salz davon zur Verwendung in der Behandlung oder Prophylaxe einer Infektionserkrankung, die verursacht ist von einem Infektionserreger, der ausgewählt ist aus pathogenen Bakterien, Pilzen und Viren, wobei das Anti-Infektionsmittel als alleiniges Anti-Infektionsmittel verwendet wird.

2. Mittel zur Verwendung nach Anspruch 1, wobei R₂ ausgewählt ist aus der Gruppe bestehend aus F, Cl, Br, I, CH₂Y, CHY₂ und CY₃, wobei Y ein Halogenatom ist.

3. Mittel zur Verwendung nach Anspruch 2, wobei R₁, R₂, R₃, R₄, R₅, R₆, R₇, R₈, R₉, R₁₀, R₁₁, R₁₃ und R₁₄ jeweils individuell ausgewählt sind aus der Gruppe bestehend aus Wasserstoff, gegebenenfalls substituiertem C₁₋₆-Alkyl und gegebenenfalls substituiertem C₁₋₆-Alkoxy.

4. Mittel zur Verwendung nach einem der vorhergehenden Ansprüche, wobei V S oder SO ist.

5. Mittel zur Verwendung nach einem der Ansprüche 1-4, wobei n eine ganze Zahl im Bereich von 1 bis 5 ist.

6. Mittel zur Verwendung nach einem der Ansprüche 1-5, wobei R₁₂ ausgewählt ist aus der Gruppe bestehend aus Wasserstoff, CH₃ und CH₂OH.

7. Mittel zur Verwendung nach Anspruch 6, wobei W zusammen mit der funktionellen Gruppe, die daran angehängt ist, CCH-(CH₂)₂-4-methylpiperazinyl, CCH-CH₂-CH(CH₃)-4-methyl-piperazinyl, CCH-(CH₂)₂-piperazinyl or CCH-CH₂-CH(CH₃)-piperazinyl ist.

8. Mittel zur Verwendung nach einem der vorhergehenden Ansprüche 1-7, wobei die Anti-Infektionsverbindung eine *trans*-Konfiguration aufweist.

9. Mittel zur Verwendung nach einem der vorhergehenden Ansprüche 1-7, wobei die Anti-Infektionsverbindung eine cis-Konfiguration aufweist.

10. Verwendung eines Mittels nach einem der vorhergehenden Ansprüche 1-9 zur Herstellung eines Arzneimittels zur Behandlung oder Prophylaxe einer Infektionserkrankung, die verursacht ist von einem Infektionserreger, der ausgewählt ist aus pathogenen Bakterien, Pilzen und Viren, wobei das Anti-Infektionsmittel als alleiniges Anti-Infektionsmittel verwendet wird.

11. Verwendung nach Anspruch 10, wobei das Mittel in einer klinisch relevanten Menge verwendet oder verabreicht wird.

12. Verwendung nach Anspruch 11, wobei das Mittel in einer klinisch relevanten Menge verwendet oder verabreicht wird, wobei eine Gleichgewichts-Serumkonzentration von weniger als 20,0 mg/l hervorgerufen wird.

13. Verwendung nach Anspruch 12, wobei die Gleichgewichts-Serumkonzentration im Intervall von 0,01 µg/l bis weniger als 20,0 mg/l ist.

14. Pharmazeutische Zusammensetzung umfassend ein Mittel wie in einem der Ansprüche 1-9 definiert und mindestens einen pharmazeutisch akzeptablen Träger oder Hilfsstoff, wobei die Zusammensetzung keine weiteren Anti-Infektionsmittel umfasst.

15. Pharmazeutische Zusammensetzung nach Anspruch 14, wobei die Zusammensetzung in einer Einheitsdosierungsform ist.

## Revendications

1. Agent anti-infectieux de la formule générale (I) 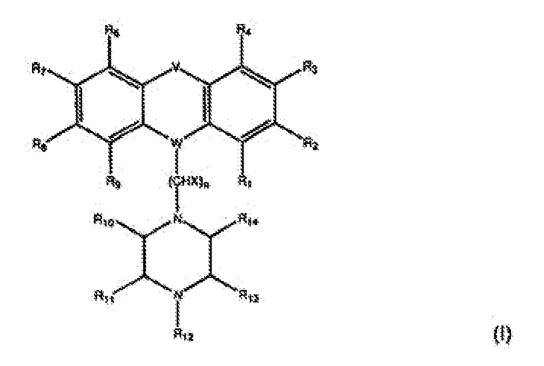 dans laquelle
W est C=CH ;
V est choisi dans le groupe constitué de S, SO₂, SO, O ou NH ;
n est un nombre entier dans l'intervalle de 1 à 6 ;
chaque X est individuellement choisi dans le groupe constitué de l'hydrogène, d'un halogène, d'un groupe hydroxy, amino, nitro, alkyle en C₁-C₆ éventuellement substitué et alcoxy en C₁-C₆ éventuellement substitué ;
R₁, R₂, R₃, R₄, R₅, R₆, R₇, R₈, R₉, R₁₀, R₁₁, R₁₃ et R₁₄ sont chacun individuellement choisis dans le groupe constitué de l'hydrogène, d'un halogène, d'un groupe hydroxy, amino, nitro, alkyle en C₁-C₆ éventuellement substitué, alcényle en C₂-C₆ éventuellement substitué, alcynyle en C₂-C₆ éventuellement substitué et alcoxy en C₁-C₆ éventuellement substitué, alcényloxy en C₂-C₆ éventuellement substitué, carboxy, alcoxycarbonyle en C₁-C₆ éventuellement substitué, alkylcarbonyle en C₁-C₆ éventuellement substitué, formyle, alkylsulfonylamino en C₁-C₆ éventuellement substitué, aryle éventuellement substitué, aryloxycarbonyle éventuellement substitué, aryloxy éventuellement substitué, arylcarbonyle éventuellement substitué, arylamino éventuellement substitué, arylsulfonylamino, hétéroaryle éventuellement substitué, hétéroaryloxycarbonyle éventuellement substitué, hétéroaryloxy éventuellement substitué, hétéroarylcarbonyle éventuellement substitué, hétéroarylamino éventuellement substitué, hétéroarylsulfonylamino, hétérocyclyle éventuellement substitué, hétérocyclyloxycarbonyle éventuellement substitué, hétérocyclyloxy éventuellement substitué, hétérocyclylcarbonyle éventuellement substitué, hétérocyclylamino éventuellement substitué, hétérocyclylsulfonylamino, mono- et di(alkyle en C₁-C₆)amino, carbamoyle, mono- et di(alkyle en C₁-C₆)aminocarbonyle, amino-(alkyle en C₁-C₆)-aminocarbonyle, mono- et di(alkyle en C₁-C₆)amino-(alkyle en C₁-C₆)-aminocarbonyle, alkylcarbonylamino en C₁-C₆, amino-(alkyle en C₁-C₆)-carbonylamino, mono- et di(alkyle en C₁-C₆)amino-(alkyle en C₁-C₆)-carbonylamino, amino-(alkyle en C₁-C₆)-amino, mono- et di(alkyle en C₁-C₆)amino-(alkyle en C₁-C₆)-amino, cyano, guanidino, carbamido, alcanoyloxy en C₁-C₆, alkylsulfonyle en C₁-C₆, alkylsulfinyle en C₁-C₆, alkylsulfonyloxy en C₁-C₆, aminosulfonyle, mono- et di(alkyle en C₁-C₆)aminosulfonyle et alkylthio en C₁-C₆ éventuellement substitué ; et
R₁₂ est l'hydrogène, un groupe hydroxy, amino, nitro, un halogène, CH₂Y, CHY₂ et CY₃, où Y est à chaque fois individuellement choisi parmi l'hydrogène, un groupe hydroxy, amino, nitro ou un halogène ;
ou son sel pour une utilisation dans le traitement ou la prophylaxie d'une maladie infectieuse occasionnée par un agent infectieux choisi parmi des bactéries pathogènes, des mycètes et des virus, dans lequel l'agent anti-infectieux est utilisé comme unique agent anti-infectieux.

2. Agent pour une utilisation selon la revendication 1, dans lequel R₂ est choisi dans le groupe constitué de F, CI, Br, I, CH₂Y, CHY₂ et CY₃, où Y est un atome d'halogène.

3. Agent pour une utilisation selon la revendication 2, dans lequel R₁, R₂, R₃, R₄, R₅, R₆, R₇, R₈, R₉, R₁₀, R₁₁, R₁₃ et R₁₄ sont chacun individuellement choisis dans le groupe constitué de l'hydrogène, d'un groupe alkyle en C₁-C₆ éventuellement substitué et d'un groupe alcoxy en C₁-C₆ éventuellement substitué.

4. Agent pour une utilisation selon l'une quelconque des revendications précédentes, dans lequel V est S ou SO.

5. Agent pour une utilisation selon l'une quelconque des revendications 1-4, dans lequel n est un nombre entier dans l'intervalle de 1 à 5.

6. Agent pour une utilisation selon l'une quelconque des revendications 1-5, dans lequel R₁₂ est choisi dans le groupe constitué de l'hydrogène, de CH₃ et de CH₂OH.

7. Agent pour une utilisation selon la revendication 6, dans lequel W est avec le groupe fonctionnel auquel il est fixé le groupe CCH-(CH₂)₂-4-méthyl-pipérazinyle, CCH-CH₂-CH(CH₃)-4-méthyl-pipérazinyle, CCH-(CH₂)₂-pipérazinyle ou CCH-CH₂-CH(CH₃)-pipérazinyle.

8. Agent pour une utilisation selon l'une quelconque des revendications précédentes 1-7, dans lequel le composé anti-infectieux présente une configuration *trans.*

9. Agent pour une utilisation selon l'une quelconque des revendications précédentes 1-7, dans lequel le composé anti-infectieux présente une configuration *cis*.

10. Utilisation d'un agent selon l'une quelconque des revendications précédentes 1-9 pour la fabrication d'un médicament destiné au traitement ou à la prophylaxie d'une maladie infectieuse occasionnée par un agent infectieux choisi parmi des bactéries pathogènes, des mycètes et des virus, dans laquelle l'agent anti-infectieux est utilisé comme unique agent anti-infectieux.

11. Utilisation selon la revendication 10, dans laquelle ledit agent est utilisé ou administré dans une quantité cliniquement applicable.

12. Utilisation selon la revendication 11, dans laquelle ledit agent est utilisé ou administré dans une quantité cliniquement applicable menant à une concentration dans le sérum à l'état stationnaire inférieure à 20,0 mg/l.

13. Utilisation selon la revendication 12, dans laquelle ladite concentration dans le sérum à l'état stationnaire se trouve dans l'intervalle de 0,01 µg/l à moins de 20,0 mg/l.

14. Composition pharmaceutique comprenant un agent selon l'une quelconque des revendications 1-9 et au moins un support ou excipient pharmaceutiquement acceptable, dans laquelle ladite composition ne comprend pas d'autres agents anti-infectieux.

15. Composition pharmaceutique selon la revendication 14, dans laquelle ladite composition est dans une forme de dosage unitaire.
